(19) Europäisches Patentamt / European Patent Office / Office européen des brevets

(11) **EP 4 423 642 B1**

(12) **EUROPEAN PATENT SPECIFICATION**

(45) Date of publication and mention of the grant of the patent:
**22.04.2026 Bulletin 2026/17**

(21) Application number: **22809468.6**

(22) Date of filing: **28.10.2022**

(51) International Patent Classification (IPC):
*G06F 21/32* (2013.01)     *G06F 21/88* (2013.01)
*H04W 12/065* (2021.01)    *H04L 9/40* (2022.01)
*A61B 5/024* (2006.01)      *A61B 5/1171* (2016.01)
*A61B 5/00* (2006.01)

(52) Cooperative Patent Classification (CPC):
**H04L 63/0861; A61B 5/0022; A61B 5/0059;
A61B 5/02438; A61B 5/1171; A61B 5/681;
A61B 5/7246; G06F 21/32; G06F 21/88;
H04W 12/065;** A61B 2503/12; G06F 2221/2139

(86) International application number:
**PCT/EP2022/080213**

(87) International publication number:
**WO 2023/073177 (04.05.2023 Gazette 2023/18)**

(54) **PROACTIVELY AUTHENTICATING USERS WITH BIOMETRIC DEVICES BASED ON PHOTOPLETHYSMOGRAMS**

PROAKTIVE AUTHENTIFIZIERUNG VON BENUTZERN MIT BIOMETRISCHEN VORRICHTUNGEN AUF BASIS VON PHOTOPLETHYSMOGRAMMEN

AUTHENTIFICATION PROACTIVE D'UTILISATEURS AVEC DES DISPOSITIFS BIOMÉTRIQUES SUR LA BASE DE PHOTOPLÉTHYSMOGRAMMES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.10.2021 CH 0704622021**

(43) Date of publication of application:
**04.09.2024 Bulletin 2024/36**

(73) Proprietor: **De Marchi, Piergiacomo**
**8044 Zürich (CH)**

(72) Inventor: **De Marchi, Piergiacomo**
**8044 Zürich (CH)**

(74) Representative: **E. Blum & Co. AG**
**Franklinturm**
**Hofwiesenstrasse 349**
**8050 Zürich (CH)**

(56) References cited:
**US-A1- 2015 135 310**

• **ZHAO TIANMING ET AL: "TrueHeart: Continuous Authentication on Wrist-worn Wearables Using PPG-based Biometrics", IEEE INFOCOM 2020 - IEEE CONFERENCE ON COMPUTER COMMUNICATIONS, IEEE, 6 July 2020 (2020-07-06), pages 30 - 39, XP033806667, DOI: 10.1109/INFOCOM41043.2020.9155526**
• **ZHAO TIANMING TZHAO7@BINGHAMTON EDU ET AL: "Your Heart Won't Lie PPG-based Continuous Authentication on Wrist-worn Wearable Devices", PROCEEDINGS OF THE 2018 ACM INTERNATIONAL CONFERENCE ON INTERACTIVE SURFACES AND SPACES, ACM, NEW YORK, NY, USA, 15 October 2018 (2018-10-15), pages 783 - 785, XP058544971, ISBN: 978-1-4503-5694-7, DOI: 10.1145/ 3241539.3267748**

**Description**

BACKGROUND

The invention relates in general to techniques (i.e., methods, devices, systems, and computer program products) of authenticating users with biometric devices, based on photoplethysmograms (PPGs). In particular, it is directed to methods allowing to proactively authenticate a user, e.g., thanks to state parameters locally stored and maintained at a biometric device, without it being necessarily needed for the device to first acquire a fresh PPG. It further relates to methods relying on machine learning-based feature extraction techniques to extract PPG features as vectors and compare such vectors to user templates, where the latter are already stored in the biometric device as reference vectors of previously extracted features.

Even though password authentication systems are convenient, they are now becoming intractable for users. The effort required (long, non-guessable passwords, composed of uppercase, lowercase, and special symbols, all different from each other, frequently changed, and never written down) is no longer reasonable, due to the increasing number of passwords needed. However, it is not possible to give up password-based security mechanisms until convincing alternatives are available, which are secure, easy to use, and easily adoptable by most users.

Different solutions to the password problem have been proposed and deployed. However, they all have drawbacks. Token solutions, like smart cards and key fobs, are more secure than passwords, but they can easily be stolen or lost. One-Time Password (OTP) alternatives do not need to be remembered by the users. However, they rely on the secrecy of the chosen pseudorandom number generator algorithm, which, if it comes to be disclosed, can expose OTPs to brute-forcing. Biometric systems, like fingerprint and face recognition, have been widely embraced for their simplicity because they do not require the users to remember anything, nor do they need to be brought along. However, they are typically non-revocable. So, if they are lost, they are lost forever with consequences that may result in identity theft.

Beyond passwords, the management of user credentials (e.g., personal identification number codes and near-field communication smart cards, such as payment and transport cards) is becoming increasingly complex. Thus, a new approach to user credential management (including passwords') is needed, which should ideally be simple to use and adopt by most users.

The document US2015135310A1 discloses a method for persistent authentication of a user, comprising: (a) sensing biometric information of a user wearing a user-wearable device, the user-wearable device including a housing that includes one or more sensors and a band that is configured to strap the housing to a portion of the user's body such that at least one of the one or more sensors included in and/or on the housing is in contact with the user's skin, and the sensing performed using at least one of the one or more sensors of the user-wearable device; (b) performing or receiving results of an authentication determination that compares the sensed biometric information to baseline biometric information to determine whether or not they match one another, wherein if the sensed biometric information matches the baseline biometric information the user wearing the user-wearable device is authenticated; (c) after determining or receiving an indication that the user wearing the user-wearable device is authenticated, monitoring whether or not the user-wearable device remains being worn by the user, the monitoring performed using at least one of the one or more sensors included in and/or on the housing and in contact with the user's skin; and (d) after determining or receiving an indication that the user wearing the user-wearable device is authenticated, for at least a period of time that the user-wearable device remains being worn by the user continually concluding that the user wearing the user-wearable device is authenticated without repeating steps (a) and (b). The sensed biometric information and the baseline biometric information comprise information related to at least one of the following: an electrocardiogram (ECG) signal; a photoplethysmography (PPG) signal; or a wrist vein pattern. ZHAO TIANMING ET AL: "TrueHeart: Continuous Authentication on Wrist-worn Wearables Using PPG-based Biometrics",IEEE INFOCOM 2020 - IEEE CONFERENCE ON COMPUTER COMMUNICATIONS, IEEE, 6 July 2020 (2020-07-06), pages 30-39 and ZHAO TIANMING TZHAO7@BINGHAMTON EDU ET AL: "Your Heart Won't Lie PPG-based Continuous Authentication on Wrist-worn Wearable Devices",PROCEEDINGS OF THE 2018 ACM INTERNATIONAL CONFERENCE ON INTERACTIVE SURFACES AND SPACES, ACM, NEW YORK, NY, USA, 15 October 2018 (2018-10-15), pages 783-785, have been cited when drawing up the European search report and examining the present European patent application.

SUMMARY

According to a first aspect, the present invention is embodied as a method of authenticating a user with a biometric device. The method comprises steps that are performed at the biometric device. First, the user is repeatedly sensed to obtain photoplethysmograms (PPGs) and verify whether at least some of the PPGs obtained match the user or not, by executing a matching procedure. Furthermore, it is repeatedly determined, based on sensor measurements, whether a condition of the user remains stable or not. In addition, a causal state parameter is repeatedly updated to set it to an unlocked state only if (i) a last verified PPG (of said PPGs) matches the user and (ii) the condition of the user is determined to have remained stable since the last successful PPG match (i.e., the time at which the last verified PPG was found to match the user). Else, the causal state parameter is set to a locked state. Accordingly, upon receiving a request originating from a computerized system to authenticate the user, the method checks a current state of the causal state parameter and responds to the request to allow the user to be authenticated only if the current state of the causal state parameter is in its unlocked state.

Thanks to the proposed approach, the user can be proactively authenticated via the biometric device when the latter receives an authentication request from a remote system. That is, the biometric device can proactively respond to the remote request without first attempting to obtain a new PPG and match this PPG, which would take too much time (i.e., more than acceptable by many contemporary users). This is made possible thanks to the fact that the causal state parameter is repeatedly updated based on outcomes of the PPG matching and user condition determination procedures, which are themselves repeatedly performed. Accordingly, if the last PPG verification led to a successful match, there is no need to try and match the user again when receiving the authentication request, provided that the condition of the user remained stable since the last successful PPG match. Thus, the proposed approach makes it possible to simplify the cumbersome and repetitive procedures required to store and enter passwords, passphrases, etc., for online activities, while maintaining security and, this, with minimal overhead in terms of time required to authenticate the user and necessary user inputs. The proposed approach further makes it possible to get rid of the numerous possessions, token credentials, etc., that a person usually carries around.

In the above method, the user is repeatedly sensed to obtain a PPG signal, and the method further comprises extracting first portions and second portions of the PPG signal. The first portions are used to form the PPGs and verify whether said at least some of the PPGs obtained match the user. The second portions of the PPG signal are used to determine whether the user condition remains stable or not, for synergy (no additional sensor is strictly needed in that case). Still, the user condition can be efficiently tracked by analysing the second signal portions.

Preferably, the biometric device is a wearable device. A wearable device is practical as the user can remain passive. This also makes it simpler to check whether the user condition remained stable. That is, determining whether said condition remains stable or not includes determining whether the biometric device is worn by the user, by executing a confirmation procedure using said second portions as input. The confirmation procedure differs from the matching procedure. The confirmation procedure can be made simpler and faster, computationally speaking, such that it can be performed more often than the verification procedure, to secure the proactive user authentication. The computer system can for example be a remote computerized system, to which the wearable device may possibly connect via an access point.

In preferred embodiments, the confirmation procedure is executed at an average frequency that is higher than an average frequency at which the matching procedure is executed. This makes it possible to compensate for the relatively long PPG matching procedure; the repeated confirmation steps enable a safe proactive verification, notwithstanding the PPG matching duration.

Preferably, the method further comprises: repeatedly updating two further state parameters, including a first state parameter and a second state parameter. The first state parameter is set to (or maintained in) a verified state or a non-verified state, depending on whether the last verified PPG matches the user or not, respectively. The second state parameter is set to (or maintained in) a confirmed state or a non-confirmed state, depending on whether the condition of the user and/or the device is determined to remain stable or not, respectively. At repeatedly updating the causal state parameter, the causal state parameter is set to (or maintained in) the unlocked state only if the first state parameter is currently in the verified state and the second state parameter was always in the confirmed state since the time at which the last verified PPG matched the user. Else, the causal state parameter is set to (or maintained in) a locked state. Such an approach institutes a simple mechanism to update the causal state parameter, which mechanism can efficiently be implemented using binary variables or Booleans.

In embodiments, the causal state parameter is immediately updated upon updating any of the first state parameter and the second state parameter, so as to be set to the locked state as soon as the first state parameter is set to the non-verified state or the second state parameter is set to the non-confirmed state. This increases the security and the efficiency of the proactive approval.

Preferably, the method further comprises continually storing values representing the sensed PPG signal in a circular memory buffer. The latter has a finite size and can only store a finite-time duration of the PPG signal sensed. The PPGs are obtained based on the stored values. Using a circular buffer limits the memory footprint of the recorded PPG signal, which is advantageous for embedded solutions. However, as the circular buffer is continually filled with fresh values, PPGs can still be continually extracted from it to continually update the state parameters.

In preferred embodiments, verifying whether a PPG matches the user comprises accessing one or more user templates that are stored on the biometric device, for security reasons. The PPG can be compared to each of the user templates accessed, to verify whether the PPG matches any one of the user templates. Several user templates can be used, which may reflect distinct user states (e.g., stressed, calm, active, sitting, etc.).

Preferably, verifying whether the PPG matches the user further comprises extracting features of the PPG as a test vector. The user templates are stored on the biometric device as reference vectors. The reference vectors were previously obtained as features extracted from reference PPGs for this user. Thus, the PPG can be compared to each of the user templates accessed by comparing the test vector with the reference vectors, e.g., by computing distances between, on the one hand, the test vector and, on the other hand, the reference vectors. Relying on vectors (already extracted for the user templates) speeds up the comparisons, while reducing the memory footprint of the user templates.

In embodiments, the method further comprises continually updating the user templates, whereby new user templates are stored in the device. The new user templates are based on selected ones of the matched PPGs. Preferably, selected user templates are deleted from the device. Thus, the time variability of PPG templates (often noted as a drawback in literature) is here leveraged to increase security and privacy. The consequences of a lost or stolen user template are less dramatic than with face pictures and fingerprints, for example.

Preferably, the user templates include several user templates. The method further comprises updating statistics based on an outcome of comparing the PPG to each of the several user templates, and the user templates are updated based on the updated statistics.

In preferred embodiments, updating the user templates comprises determining, based on the updated statistics, whether to store the PPG as a new user template or not, and, in the affirmative, storing a representation of the PPG as a new template in the device.

Preferably, updating the user templates comprises executing a garbage collection algorithm using the updated statistics as input, to delete one or more of the user templates as currently stored in the device.

In embodiments, the computerized system is a remote computerized system, and the request is received and responded to by the device in accordance with one or more authentication protocols of one or more specifications and/or methods, e.g., a protocol of a logical authentication specification such as involved in the so-called FIDO2 set of specifications developed by the Fast IDentity Online (FIDO) Alliance or a protocol of a multi-factor authentication method, or a protocol of a physical authentication specification such as involved in the Federal Information Processing Standard 201 (FIPS 201) standard.

In embodiments, for each PPG of at least some of the PPGs obtained, the method extracts features of the PPG as a test vector and verifies whether the test vector matches a user template, by accessing one or more user templates and comparing the test vector with the one or more user templates accessed. The one or more user templates accessed are stored in the biometric device as one or more reference vectors of features previously extracted from one or more reference PPGs for this user, respectively. So, upon receiving a request originating from a remote computerized system to authenticate the user, the device can respond to the request according to an outcome of verifying whether the test vector matches a user template. As noted earlier, relying on vectors of extracted features enables a quick verification and reduces the memory footprint of the user templates.

In preferred embodiments, each PPG is obtained as a timeseries, and the features are extracted from each PPG (the latter preferably of a predefined length) using a pretrained extractor. This extractor is implemented by an artificial neural network (ANN), which preferably includes convolutional neural network (CNN) layers and recurrent neural network (RNN) layers. The extracted vectors are m-dimensional vectors, where $m \geq 32$ and, preferably, $m = 64$ or $128$. All the vectors are normalized to a same reference length, to enable efficient comparisons. A key advantage of using a pre-trained ANN as an extractor only is that a same extractor can be obtained, which is the same for all potential users: the model generalizes well to previously unseen individuals. No training is required at the biometric device; the extractor can be trained (and possibly retrained) at an external computer, which may leverage online mining and batching strategies.

In embodiments, weights of each of the CNN layers and the RNN layers are quantized according to an $m$-bit quantization scheme, where $m \leq 32$ and, preferably, $m = 8$. The ANN weights can be quantized prior to transferring the ANN parameters to the biometric device, to speed up inference at runtime on the device as well as to reduce memory and power consumption on the device. The ANN, as implemented by the biometric device, is preferably free of any bias coefficient, be it as a result of the quantization.

In embodiments, the method further comprises, prior to repeatedly obtaining the PPGs at the biometric device, training an initial extractor at an external computer, thanks to an $n$-uplet loss algorithm, where $n = 3$ or $4$. The $n$-uplet loss algorithm is trained according to $n$-tuples, each involving at least one valid PPG and at least one invalid PPG for a respective user, to obtain trained parameters for the initial extractor. The trained parameters are then transferred to the biometric device, and stored therein, with a view to subsequently running the extractor at the biometric device. The trained weights are preferably quantized according to an $m$-bit quantization scheme, where $m \leq 32$, prior to transferring them to the biometric device. Using $n$-uplet loss algorithms at training was found to markedly increase the performance of the subsequent feature extractions and verifications at the biometric device.

In embodiments, the one or more user templates comprises several templates, the test vector is compared with each of the several templates using a distance metric to obtain distances, based on which it is verified whether the test vector matches the user. The distance metric is preferably based on a Euclidean distance. A classification based on distance computations is computationally more efficient, and thus quicker than classifications performed thanks to statistical models or cognitive models (i.e., including inferencing layers). All the more, this is much more practical in the context of PPGs as user templates can easily be updated, frequently, without having to re-parameterize or re-train the cognitive model, which is here used for feature extraction only, not inferencing.

In preferred embodiments, the method further comprises updating statistics based on said distances and updating the templates stored in the biometric device based on the updated statistics.

Preferably, the one or more user templates are stored encrypted or obfuscated, and the method further comprises decrypting or de-obfuscating the templates prior to verifying the templates.

In embodiments, each PPG is obtained by acquiring a PPG signal, detecting a systolic peak in the PPG signal acquired, and extracting a signal segment centred on the detected systolic peak. The signal segment has a predetermined length, which turns out to markedly improve the results in practice.

Preferably, each PPG is obtained by: acquiring a PPG signal; sampling the PPG signal acquired at an average sampling frequency that is between 5 and 7500 Hz, to obtain values representing the PPG signal; and storing said values in the device according to a $l$-bit resolution, where $8 \leq l \leq 32$.

According to another aspect, the invention is embodied as a biometric system comprising a biometric device, where the biometric device comprises: a sensing unit configured to acquire PPG signals; an interface configured to connect the biometric device to a computerized system (preferably a remote computerized system, more preferably via a network access point); and a processing unit configured to take steps according to any of the methods evoked above.

According to a final aspect, the invention is embodied as a computer program product for authenticating a user with a biometric device. The computer program product comprises a computer readable storage medium having program instructions embodied therewith. The program instructions are executable by processing means of the biometric device to cause the latter to take steps according to any of the methods evoked above.

BRIEF DESCRIPTION OF THE DRAWINGS

These and other objects, features and advantages of the present invention will become apparent from the following detailed description of illustrative embodiments thereof, which is to be read in connection with the accompanying drawings. The illustrations are for clarity in facilitating one skilled in the art in understanding the invention in conjunction with the detailed description. In the drawings:

FIGS. 1A and 1B depict examples of wearable biometric devices, as involved in embodiments. FIG. 1A shows a smartwatch, while FIG. 1B depicts a ring;

FIG. 2 depicts a biometric system in which a biometric device (such as shown in FIG. 1A) interacts with a remote server via an access point configured at a personal computer, as in embodiments;

FIG. 3 is a diagram that schematically represents components of a biometric device, as involved in embodiments of the invention;

FIG. 4 schematically illustrates the operation of a biometric device configured to obtain photoplethysmograms (PPGs) in transmission (FIG. 4A) or in reflexion (FIG. 4B), as in embodiments;

FIGS. 5A and 5B are plots representing PPG signal chunks, from which features are extracted with a view to authenticating a user, as in embodiments;

FIGS. 6A, 6B, 6C, 8, and 9 are flowcharts illustrating high-level steps of methods of authenticating a user with a biometric device, according to embodiments;

FIG. 7 is a state diagram illustrating how a biometric device behaves as a finite-state machine, as in embodiments;

FIG. 10A schematically illustrates the training of a feature extractor thanks to a triplet loss algorithm, where the algorithm is trained according to valid and invalid PPGs for a respective user, as in embodiments; and

FIG. 10B is a flowchart illustrating the training of the feature extractor, the quantization of its weights, prior to transferring the extractor parameters to a biometric device, as in embodiments.

The accompanying drawings show simplified representations of devices or parts thereof, as involved in embodiments. Technical features depicted in the drawings are not necessarily to scale. Similar or functionally similar elements in the figures have been allocated the same numeral references, unless otherwise indicated.

Biometric systems, methods, and computer program products embodying the present invention will now be described, by way of non-limiting examples.

DETAILED DESCRIPTION OF EMBODIMENTS OF THE INVENTION

The following description is structured as follows. General embodiments and high-level variants are described in section 1. Section 2 addresses more specific embodiments and technical implementation details.

The proposed methods and their variants are collectively referred to as the "present methods". All references Sn refer to methods steps shown in the flowcharts, while numeral references pertain to physical parts or components of biometric devices and systems.

### 1. General embodiments and high-level variants

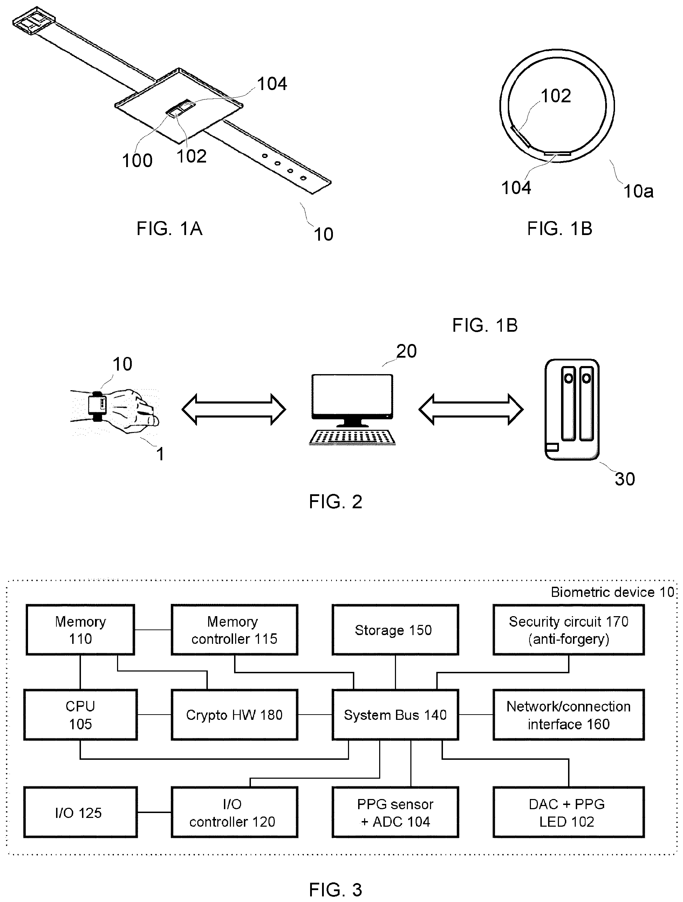

In reference to FIGS. 1 - 7, a first aspect of the invention is described, which concerns a method of authenticating a user with a biometric device 10. The method comprises a series of steps that are performed at the biometric device 10. The latter may notably form part of a portable device such as a smartphone or a tablet. Preferably, however, the device 10 is designed as a wearable device 10, such as a smart watch (as assumed in FIG. 1A) or a ring (as in FIG. 1B). Other types of biometric devices can further be contemplated. The device 10 should be able to communicate with a computerized system 20, 30, e.g., a remote system 30, via an access point, which may for instance be configured at a device 20, e.g., a companion device or a device such as a laptop or a smartphone of the user 1, which can be configured as a client device of the remote system 30. The device 10 actually concerns another aspect of the invention (a biometric system), which is described later in detail.

The device 10 is notably configured to produce photoplethysmograms (PPGs), which are exploited to authenticate the user, albeit in an indirect manner, so as to proactively respond to remote authentication requests. That is, the method relies on PPG signals obtained or segments of such signals. The PPG signals exploited may be basic PPG signals, possibly transformed into n-derivative PPG signals, where, e.g., $n = 1$ or 2. Note, the acronym PPG denotes a photoplethysmogram, which is a representation (e.g., a digital, sampled representation) of the initial signal obtained by a PPG sensor 100. The word "photoplethysmograph" refers to the instrument (here the device 10) used to obtain the PPG, while "photoplethysmography" generally refers to the underlying sensing technique. In this description, the acronyms "PPG" or "PPGs", when used alone, refer to a certain digital representation (or representations) of the signals or segments thereof. When used in combination with other words, terminologies such as "PPG signal" or "PPG sensor" refer to concepts (e.g., signal, sensor) relating to photoplethysmography.

The PPG verification mechanisms proposed herein typically rely on representations of PPG signal segments. A PPG signal segment refers to a signal portion that typically includes at least one signal period (in fact, a quasi-period) or multiple signal periods (or quasi-periods).

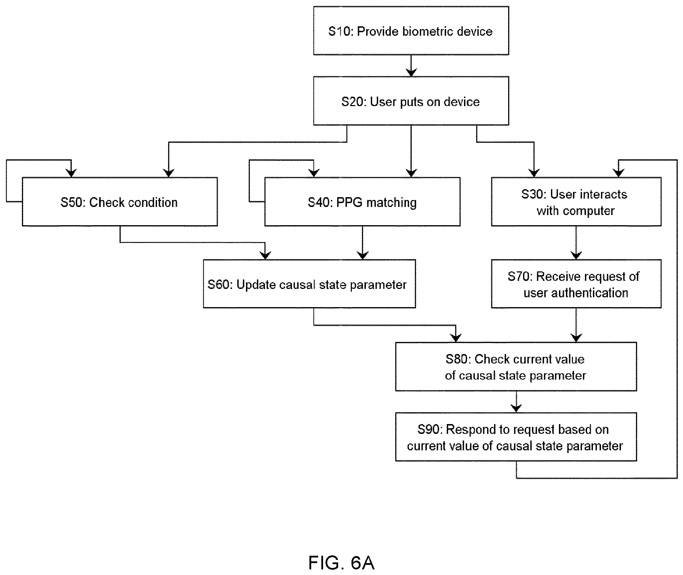

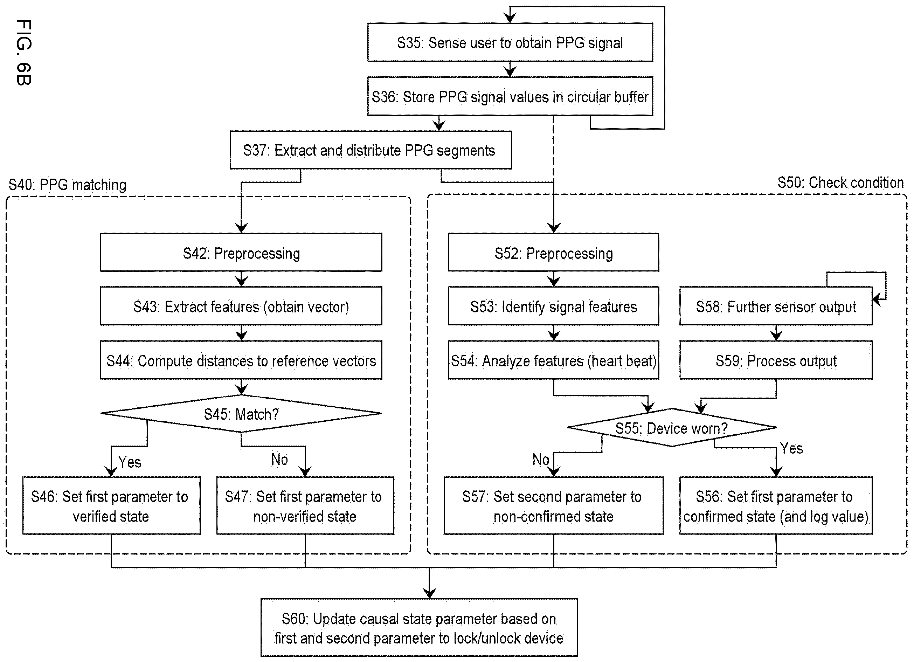

According to the proposed method, the user is repeatedly sensed S35 to obtain S37 PPGs, i.e., digital representations of PPG signals or segments thereof, as illustrated in FIG. 6B. Then, the method verifies whether at least some of the PPGs obtained match S45 the user or not, by executing S40 a matching procedure, see FIGS. 6A and 6B. This procedure typically amounts to comparing a PPG to user templates. Various examples of suitable matching procedures are discussed later in detail. Note, the user 1 is normally a human user, although the present techniques may, in principle, apply to animals too.

Aside from obtaining PPGs and verifying such PPGs, the method repeatedly determines S50, S55 whether a condition of the user 1 remains stable or not, based on some sensor measurements. The "condition" of the user relates to the state of the user and/or the environment of the user (including the device 10) or, more generally, to circumstances that may potentially impact the extent to which remote authentication requests may be approved for user authentication or not. The user condition may notably be assessed by sensing whether the device 10 was uninterruptedly worn since the last successful PPG match, should the biometric device 10 be a wearable device, as in preferred embodiments. The sensor measurements may advantageously exploit PPG signals or parts thereof, as in preferred embodiments. In fact, the verification step S50 solely relies on PPG signal segments to confirm the condition of the user. However, the verification S50 typically uses a procedure that is distinct from the matching procedure. In variants, the verification performed at step S50 may additionally involve other types of signals, such as capacitive signals, inertial measurement unit (IMU) signals, audio signals, and/or video signals.

Moreover, the method repeatedly updates S60 a causal state parameter, so as to set this parameter to (or maintain it in) one of two state parameters, reflecting a locked state and an unlocked state of the device. Namely, the causal state parameter is set to (or maintained in) an unlocked state only if: (i) the last PPG that was verified was found to match the user; and (ii) the condition of the user is determined to have remained stable since the last successful PPG match, i.e., since the time at which the last verified PPG was found to match the user. Else, the causal state parameter is set to (or maintained in) a locked state.

The causal state parameter is used by the device to respond to authentication requests from a computer system, where such requests aim at authenticating the user 1. Such requests typically originate from an external computer system 20, 30 and are accordingly sometimes referred to as "remote authentication requests", "remote requests", or simply "requests", in this document. When the device 10 receives S70 a remote request, the device 10 first checks S80 the current state of the causal state parameter and then responds S90 to the request to allow the user to be authenticated only if the current state of the causal state parameter is in its unlocked state.

So, the proposed method aims at authenticating a user, with a view to authorizing the user 1 to, e.g., access a service, a platform, a website, perform an online transaction or a contactless payment, and/or gain access to a physical location. In such cases, the remote requests typically originate from remote systems 30 such as servers; they are typically routed through an access point configured at a standard computer device 20, which typically is a user device, such as a laptop, a table, or a smartphone.

A remote request can be received S70 and responded S90 to in accordance with one or more authentication protocols. Any suitable authentication protocol can be contemplated, such as a protocol matching specifications developed by the Fast IDentity Online (FIDO) Alliance and the World Wide Web Consortium (W3C). Interactions with remote systems 30 may for instance be based on the FIDO2 set of specifications, see https://fidoalliance.org/fido2/, to enable password-less solutions. In particular, a password-less login flow can be enabled based on the so-called Client to Authenticator Protocol (CTAP) and Web Authentication (WebAuthn). Moreover, the present approach can be exploited to provide a second factor, as in the so-called Two Factor Authentication method. This approach can also be used for access rights, e.g., to substitute a badge to access a building. Such applications may for instance involve a protocol from a physical authentication specification such as involved in the Federal Information Processing Standard 201 (FIPS 201).

In variants, the method is exploited to enable a local authentication, e.g., authenticate a user when the latter switches on a tablet or smartphone, or when the user wants to gain access to a physical location through a card reader, for example. Such applications do typically not require an access point. A local authentication mechanism is involved, inasmuch as the device (tablet, smartphone, etc.) requesting the authentication is physically close to the biometric device 10 that enables the authentication. Again, various protocols can be contemplated.

In further variants, the method is exploited to enable an on-device authentication, e.g., authenticate a user when the latter puts on a smartwatch. In that case, the authentication request originates from the wearable device itself and the wearable device needs to authenticate the user.

The authentication mechanism enabled by the present approach first involves a local verification process S40, based on PPGs, whereby the biometric device 10 locally verifies, repeatedly, that the PPGs (or, at least, some of them) match the user. The second step S50 reinforces security as it further assesses whether a situational change has occurred, which would require a new PPG check, thus preventing fraudulent (or otherwise inappropriate) uses. E.g., the second step S50 may oblige the user to continuously wear the device 10, should the latter be a wearable device, as in embodiments. All the more, the second step S50 makes it possible to proactively respond to remote authentication requests, as discussed below in detail.

The two steps S40, S50 lead to update the causal state parameter, which governs the extent to which the user can be authenticated remotely or locally or on device, depending on the use case as discussed above. Accordingly, the user can be proactively authenticated when the biometric device 10 receives an authentication request. I.e., the biometric device 10 can proactively respond to the remote request without first attempting to obtain a new PPG and match this PPG to the user, which would take too much time (i.e., more than acceptable by many contemporary users). This is made possible thanks to the fact that the causal state parameter is repeatedly updated based on outcomes of steps S40, S50, which are themselves repeatedly performed. Accordingly, if the last PPG verification led to a match, there is no need to try and match the user again upon receiving the request, as long as the outcome of step S50 indicates that the condition of the user remained stable since the last successful PPG match (e.g., the device 10 was continuously worn since the last PPG match).

Note, the device 10 does not need to systematically respond to remote requests S70. If the device 10 is locked, then it may simply ignore the remote request. Still, the device 10 may possibly respond to this request, but it will not instruct or take steps to allow the user to be authenticated. That is, the device 10 may systematically respond S90 to such requests, by indicating that the user is verified (and thus can be authenticated) or that the user is not verified (in which case the user cannot be remotely authenticated).

Thus, the proposed approach makes it possible to simplify the cumbersome and repetitive procedures required to store and enter passwords, passphrases, etc., for online activities, while maintaining security and, this, with minimal overhead in terms of time required to authenticate the user and user inputs. Still, the present methods will normally ensure the user's willingness to be authenticated. The proposed approach also makes it possible to get rid of the numerous possessions, token credentials (e.g., smart cards, USB sticks, car, house keys, etc.) that a person usually needs to be authenticated, while maintaining security with minimal overhead and user inputs.

Comments are in order. To start with, steps S35 (sensing), S50 (user condition), and S60 (causal state parameter) are repeatedly performed, meaning that such steps are continually performed (e.g., during working hours or when the user is active, digitally), though not necessarily at regular time intervals. Note, the matching procedure may possibly be performed only once during a same session, whereas the confirmation procedure S50 will typically be performed several times after a matching procedure S40, hence causing repeated updates of the causal state parameter. In all cases, the steps S35, S40, S50, and S60 are meant to be repeatedly performed over days and weeks and the user will typically need to be repeatedly authenticated. In addition, such steps are typically performed concomitantly, despite interdependences (i.e., step S60 depends on outcomes of both steps S35 and S50, and step S50 may possibly be triggered dependent on the timing of the last successful PPG match).

Besides, continual interactions between the biometric device 10 and remote systems 30 are typically mediated via an access point 20 configured at a laptop, a smartphone, or a tablet. Thus, the present approach may be used to enable a two (or more) factor authentication without requiring repeated user inputs. E.g., the user just needs to wear the device 10 or somehow remain in a stable condition (as assessed by the device 10), and also demonstrate willingness to authenticate. Thus, the user can be repeatedly authenticated with minimal constraints.

The device 10 may possibly interact with remote systems 30 and access points 20, e.g., thanks to wireless interface protocols, such as Bluetooth Low Energy (BLE) and Near-field communication (NFC) protocols. The remote systems 30 and access points 20 may possibly monitor the user, such that access rights can be revoked should the legitimacy of the user be put in question (e.g., the user moves away from the access point 20). This may notably be used to increase security of remote systems over open sessions.

The present authentication processes must be distinguished from a mere identification, an identity validation, and an identity verification process. An authentication is the process by which an individual's identity is qualified against something that only this individual should know or have, here a PPG pattern, together with a biometric device 10 and, possibly, another user device 20. This type of authentication can be regarded as a multi-factor authentication, given that the user possesses the biometric device 10 and has certain PPG patterns. The proposed approach can also be regarded as a local user verification, which, in turn, enables a user authentication (e.g., at a remote website), thanks to a suitable protocol or specification.

Still, the local verification S40 enabled by the biometric device may possibly involve (and/or be complemented by) strong authentications, i.e., based on challenge-response and multi-factor. That is, the device 10 may possibly respond to a challenge from the remote computer 30 according to a pre-established protocol, e.g., based on a key, as in the FIDO2 set of specifications. This, however, is preconfigured and does not require user inputs at runtime. In addition, the local verification steps performed by the device 10 can be preceded by one or more authentication steps, e.g., to initially enrol the user 1 at the device 10 and/or at a website to which the user connects to initialize the device 10.

The first routine S40 typically requires some pre-processing S42. The PPGs exploited may further be subjected to some verification as to the quality of signal acquired. Thus, not all the PPG signal segments may effectively be exploited. Additional checks may be carried out in respect of the PPG segments, the device 10 and/or the user 1, to assess a current state of the user. E.g., is the user resting, running, etc.? This can be achieved by analysing the PPG signal itself, or segments thereof, IMU signals, and/or other signals, as discussed later. These additional checks may, in turn, impact the matching procedure. E.g., the matching procedure may compare a PPG with user templates selected in accordance with the current state of the user.

The first routine S40 is used to check the PPG signals. The matching procedure is repeatedly executed for each, or at least some, of the PPGs, to verify the user. This procedure may for example involve a statistical model or, better, a trained model. Preferably, it involves a suitably trained feature extractor (i.e., involving neural layers), as well as valid user templates stored in the device 10 in the form of reference vectors of extracted features. Such an approach was found optimal in terms of runtime computational efforts, memory footprint, and false positives.

The second routine S50 uses PPG signals, too. Such signals are analysed to check whether the condition of the user remains stable. The goal is to assess whether the user condition remained sufficiently stable (e.g., whether the device was continuously worn) since the last successful PPG match, so that it is possible to rely on the last successful match with sufficient confidence. In preferred embodiments, the second routine S50 only exploits PPG signals, for synergy. Thus, step S50 may leverage the same PPG circuit as step S40, albeit using a simpler and faster procedure. Thus, the confirmation may be repeatedly performed at shorter time intervals.

Again, some pre-processing S52 may be required, especially if the second routine exploits PPG signals too, as assumed in FIG. 6B. Note, such pre-processing steps may possibly be performed upstream both routines S40, S50 (contrary to the assumption made in FIG. 6B). In that case, each routine benefit from a same type of pre-processing. Preferred, however, is to rely on separate pre-processing steps S42, S52, as the pre-processing required for the routine S40 may be more demanding, computationally speaking.

The processing time required to check S50 the user condition is typically faster, even when it relies on PPG signal. Thus, the frequency at which the second routine S50 is performed can be (and preferably is) larger than the frequency at which the first routine S40 is performed. The second frequency may for instance be between 1 Hz and 10 Hz, while the first frequency may for instance be between 0.0017 Hz and 0.0333 Hz. The second frequency should be sufficiently high to detect a quick change in the user condition, e.g., when the device is quickly removed from the user and placed on a different user.

The present method exploits segments of the PPG signals in order to locally verify both the user S40 and its condition S50. Referring to FIG. 6B, the user can be repeatedly sensed S35 to obtain S36 a PPG signal. Then, first portions (i.e., chunks) of the PPG signal are extracted S37 to form said PPGs and verify whether at least some of the PPGs obtained match S45 the user. Second portions (distinct chunks) of the PPG signal are extracted S37, based on which it is determined S52 - S55 whether the user condition remains stable or not. Yet, steps S52 - S55 typically rely on a procedure that is distinct from and simpler than the matching procedure S40, as noted earlier. Note, the method may possibly adapt, dynamically, the number or length of the first portions relative to the number or length of the second portions, based on an outcome of the matching procedure. That is, the algorithm may change the frequency at which PPGs are acquired based on the outcome of the PPG checks. For example, the user may, by default, be sensed at fixed time intervals but a heuristic may be used to modify the points in time at which the user is sensed based on the comparison outcomes. E.g., if the agreement between fresh PPGs with user templates tend to deteriorate over time, then it may be useful to increase the sensing frequency, with a view to maintaining sufficient certainty as to the user and/or updating the user templates used for comparisons.

In preferred embodiments, the biometric device 10 is a wearable device, such as a smartwatch (FIG. 1A) or a ring (FIG. 1B). In variants, the device 10 may be inserted in (or otherwise form part of) a garment, such as a brassiere or an armband. In such cases, the routine S50 aims at determining S55 whether the biometric device 10 is being worn by the user 1. Note, sensing whether the biometric device 10 is being worn may equivalently be achieved by sensing whether the biometric device is not being worn, this depending on the detection means used. Step S50 involves an execution S52 - S55 of a confirmation procedure, which may possibly exploit PPG signals too, as noted above. Still, the confirmation procedure S50 typically differs from the matching procedure S40.

Like the matching procedure S40 and other procedures involved herein, the confirmation procedure S50 is typically stored in the device 10. E.g., such procedures can be loaded in the main memory of the device 10 for subsequent execution by processing means 105 of the device 10. In variants, such procedures are hardcoded. However, a different algorithm can be used to verify S50 the user condition, even where it exploits PPG signal segments obtained via the same PPG sensor 100. The confirmation procedure can typically be made simpler than the user verification procedure because the criteria required to confirm the user condition are less stringent. Reusing PPG signals, also for the user confirmation procedure, is appealing, because a single sensor (a PPG sensor) is required in that case.

In variants, however, the confirmation procedure relies on another type of sensor, or several types of sensors, e.g., an accelerometer, a gyroscope, a magnetometer, a proximity sensor possibly a capacitive one, a microphone, a camera, a temperature sensor, and/or a body impedance sensor, as included in the device 10. Note, in practice, the use of such sensors may require preapproval by the user, especially if such sensors are activated in a smartphone. In other variants, multiple PPG sensors can be involved.

For example, one possible way to assess whether the device 10 is being worn is to use a combination of sensor signals such as signals obtained from a proximity sensor possibly a capacitive sensor, the PPG sensor, and/or an anti-forgery mechanism. A proximity sensor can sense changes related to the surface where the device lies. If the proximity sensor is based on capacitive technology, it can distinguish whether such a surface is living skin or a table, for example. In addition, one may check whether the user heart rate (HR) lies in an admissible range and whether it does not change abruptly, based on the PPG signals. An anti-forgery mechanism can be used to detect if the device 10 is forced (e.g., cut from someone's wrist). Such a mechanism can for instance use an electric wire running around the circumference of a bracelet or a ring. If this wire gets cut, the device 10 locks. A further type of sensor is an IMU system to detect accelerations or movements of the device 10. Various heuristics can be devised to analyse outputs produced by such sensors and mechanisms and conclude as to whether the condition of the user remained stable or not.

In embodiments, the present approach relies on multiple state parameters, including a first state parameter and a second state parameter, in addition to the causal state parameter. The first state parameter and the second state parameter reflects outcomes of the PPG verification S40 and the user condition determination S50. That is, the first state parameter is set S46, S47 to a verified state S46 or a non-verified state S47, depending on whether the last verified PPG matches S45 the user or not, respectively. Similarly, the second state parameter is set S56, S57 to a confirmed state S56 or a non-confirmed state S57, depending on whether the condition of the user and/or the device is determined S55 to remain stable or not, respectively. This institutes a simple mechanism to update S60 the causal state parameter. I.e., the latter is set to the unlocked state only if the first state parameter is currently in the verified state and the second state parameter was always in the confirmed state since the last successful PPG match. Else, the causal state parameter is set to the locked state.

The above state parameters are maintained at the device 10, i.e., continually updated based on outcomes of the routines S40, S50. Note, such state parameters represent model states and are frequently updated, such that they are effectively variables. To set a state parameter to a given state means storing a value indicative of that given state. The first parameter value indicates whether the last PPG was successfully matched to the user. The procedure S40 or S60 may possibly involve a countdown timer, whereby the device 10 locks if the user happens not to be verified for a long time. The countdown is reset at the next successful PPG match.

The second state parameter value may for instance indicate whether the device was or is currently worn by the user, e.g., whether the last measurement gave rise to conclude that the device was still being worn at the time of this measurement. Where multiple techniques are used to detect if the device is being worn, the second state parameter value can be determined thanks to simple AND operations between outcomes of such techniques.

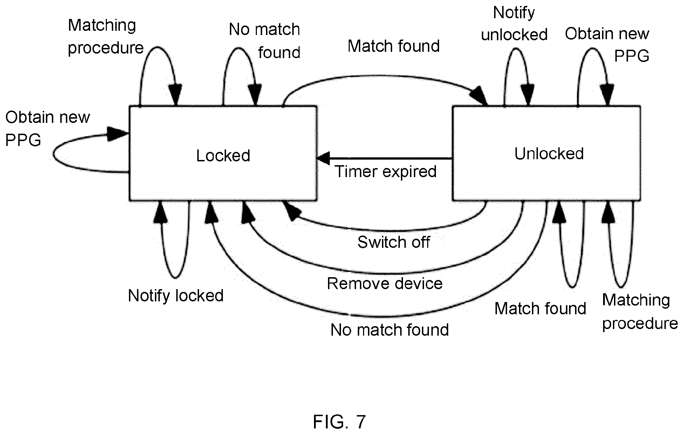

The causal state parameter acts as a global verification parameter, which is set to a locked state if (and preferably as soon as) any of the first and second state parameters is negative (non-verification or non-confirmation). It can only be set to its unlocked state if both the first and second state parameters indicate a success (verification and confirmation). That is, the causal state parameter is built according to values taken by the first and second state parameters, in such a manner that a user cannot be remotely authenticated, notably if the device 10 is switched off, removed, forced (or somehow the user condition oddly changes), or if the last PPG verification failed, as illustrated in the state diagram of FIG. 7.

For example, the first parameter may be set to "1" if the last PPG is successfully matched to the user, else it is set to "0". Similarly, the second parameter may be set to "1" and maintained to such a value as long as the user condition is determined to remain stable (e.g., the device is determined to be worn), else to "0". Successive values of the second state parameter (as obtained since the last PPG match) may for instance be logged, such that it suffices to multiply the first state parameter value by all of the logged values to obtain the causal state parameter value. That is, the second parameter value may be logged to form an array of successive values. This way, the causal state parameter can be obtained (when needed, e.g., upon receiving S70 a remote request), by multiplying the current value of the first parameter by all the successive values obtained for the second parameter since the last PPG match. This results in a "1" if the device was uninterruptedly worn since the last PPG match or a "0" if an interruption was detected or if the last PPG check failed.

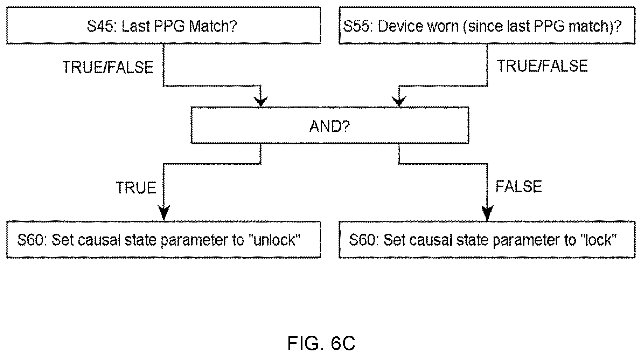

A more efficient algorithm is the following. A change of value of any of the first and second state parameters may immediately cause to lock the device. That is, the causal state parameter is preferably updated, systematically, after each change in the first or second parameter value. For example, the causal state parameter value may be initialized to the last known value of the first parameter ("0" or "1") and then repeatedly multiplied by the last value obtained for the second parameter. In that case, there is no need to log successive values of the second parameter, as in preferred embodiments. A similar result can be achieved thanks to AND tests applied to values (captured as Booleans) obtained in output of steps S45 and S55, as illustrated in FIG. 6C.

Other heuristics can similarly be devised, which have a negligible computational cost. For instance, the whole process S40 - S60 can be implemented in a while TRUE loop, where the causal state parameter is updated at the end of each loop, based on the last known parameter value of each of the first and second state parameters, independently of whether such parameters were updated or not during the last loop. Two variables are stored across time to remember the states of the two routines. Each of the two state parameters is initially initialized, e.g., to "0" or FALSE, corresponding to "not verified" and "not confirmed", respectively.

Additional state parameters may possibly be involved, e.g., including a parameter reflecting a willingness of the user to be authenticated. This parameter is updated based on user inputs. Such a user parameter may for instance be set *a priori,* possibly by default. This, however, may introduce security vulnerabilities and be incompatible with some user authentication specifications. Thus, the user parameter is preferably set upon receiving S70 each remote authentication request, for the user to approve each request. This is desirable where the user wishes to keep control over each authorization, e.g., to prevent tap-and-go fraud. Note, the various procedures S40 - S60 are preferably put on hold when the user indicates s/he does not wish to be authenticated.

Notwithstanding the above examples, the first and second parameters need not necessarily be binary parameters. Continuous parameter values may for instance be stored, which can use one or more threshold values delimiting a non-verified or non-confirmed state from a verified or confirmed state. In other variants, only one of the first two parameters may be a binary parameter, while the other may take continuous values. Such nonbinary values may further be used to assess the need for modifying parameters used to perform steps S40 and S50. E.g., if the value of the first parameter is only slightly above the threshold required for the user to qualify as a verified user, then the device 10 may come to more frequently verify the user, to maintain certainty. An update and/or garbage collection mechanism may similarly benefit from such thresholds.

As noted above, the causal state parameter is preferably immediately updated S60 upon updating S46, S47, S56, S57 any of the first state parameter and the second state parameter. This way, the causal state parameter is set S60 to the locked state as soon as the first state parameter is set to the non-verified state or the second state parameter is set to the non-confirmed state. Such a mechanism institutes an exit condition (similar to an interrupt), which locks the device if and as soon as any of the two state parameter values denotes a non-verified or non-confirmed state (this is an OR condition). So, the device 10 will immediately prevent the user 1 to be authenticated as soon as any of steps S40, S50 fails. Conversely, the device may immediately set the causal state parameter to the unlocked state as soon as both the first state parameter and the second state parameter are set to the verified and confirmed states again.

The above mechanism can be implemented with loops including proper exit conditions. In variants, it is implemented by true interrupts, i.e., thanks to interrupt events and/or interrupt handlers, having priority over other routines and procedures. In that cases, the use of first and second parameters is superfluous as interrupts may directly modify the value of the causal state parameter. For example, a capacitive proximity sensor can trigger an interrupt as soon as it detects that the device 10 is no longer in touch with living skin. Such an interrupt may not only modify the second state parameter but also directly modify the causal state parameter. Each interrupt can be implemented in hardware and/or software. An interrupt event can be triggered by a software or hardware event. An interrupt handler is code that is executed by the central processing unit (CPU) upon suspending its current activities. Interrupts are associated with priority levels, which determine the order in which to serve multiple concurrent interrupts, and whether or not to serve them.

Combinations of interrupts and state parameters can be contemplated too. For example, a loop (with an exit condition) can be used to continually check the PPGs, while interrupts can be used to verify whether the device is still being worn, notably with a capacitive sensor, an anti-forgery mechanism, etc.

In the scenarios contemplated herein, the confirmation procedure S50 is typically simpler and thus executes faster than the matching procedure S40. Thus, it is preferably executed more often (i.e., at a higher average frequency) than the matching procedure, as noted earlier. This makes it possible to compensate for the relatively long PPG matching procedure. Repeated confirmation steps S50 can accordingly take place between two successive PPG verification steps S40, which enables a safe proactive verification, notwithstanding the PPG verification duration. Note, if the same PPG sensor 100 is used for both routines S40, S50, then the device 10 will typically not be able to concurrently verify the user and its condition. However, the algorithm may advantageously leverage the result of the last PPG check to accordingly update the second state parameter. I.e., a successful PPG match indicates that the device is still being worn by the user. Conversely, the PPG verification may indicate that the device is still being worn, even if it failed to successfully match the user.

Embodiments involve a circular memory buffer, in which the PPG signal is stored and continually renewed. More precisely, the method may continually store S36 values representing the sensed PPG signal in the circular memory buffer. The latter stores a finite time duration of the PPG signal sensed. The memory limitation of the circular buffer results in continually rewriting to the buffer. Yet, PPGs can still be continually extracted S37 from the buffer to continually update the state parameters. The circular buffer may for example store 30s of continuous signal. Segments of 1.5s to 4s are typically extracted. E.g., a first segment of 4s may be exploited to verify S40 the user, while remaining segments (e.g., of 2s each) may be exploited to check whether the user condition remained stable S50. In variants, the confirmation procedure may analyse the user HR over the full signal as stored in the whole buffer (i.e., by reusing the first portions of the PPG signal, in addition to the second portions thereof).

In embodiments, the matching procedure S40 relies on one or more user templates, which are stored on the biometric device 10, for security reasons. Preferably, several user templates are stored on the device 10, as assumed in the following. The matching procedure S40 may access S44 the user templates (as initially stored on the device 10) and compare S44 a PPG to each of the user templates accessed, to verify whether this PPG matches S45 any of the user templates. Preferably, the present methods involve a feature extraction algorithm (in a machine learning sense) and the user templates are directly stored as vectors in the device 10, to speed up the comparison. That is, the verification step S40 comprises extracting S43 features of a PPG as a test vector, while the user templates are already stored on the biometric device 10 as reference vectors; these have been previously obtained as features extracted from reference PPGs for this user 1. In that case, the verification can easily be achieved by comparing S44 each test vector with the reference vectors, e.g., by computing distances between the test vector and each of the reference vectors. In other words, a PPG is verified by comparing a representation thereof (i.e., a vector of extracted features) with each reference vector as previously extracted from valid PPGs. Note, this comparison may take into account the current state of the user, so as to select reference vectors corresponding to a same user state (e.g., calm, stressed, sitting, walking, running, etc.). Indeed, several user templates may possibly be acquired (and updated), to account for different potential user states, as noted earlier.

Any suitable distance metrics can be used for the comparison, based on which the verification can easily be completed. Comparing vectors based on extracted features is computationally more efficient than performing full inferences (classifications, predictions) with a cognitive model. Still, the feature extraction is preferably performed using a trained artificial neural network (ANN). However, this ANN is only used to extract features and does not directly produce inferences. I.e., it does not need additional neural layers to directly produce inferences. Rather, this ANN is used to produce vectors, based on which comparisons are made (distances are computed), which allows a conclusion to be drawn. In addition, a solution relying on vectors of extracted features lends itself well to user template updates and, thus, allows the time variability of PPG signals to be better accounted for. Note, feature extraction is described later in detail.

In less preferred variants, the device 10 uses a statistical or a cognitive model designed so as directly lead to a classification result (verified or non-verified), or a prediction result (a score interpreted as a verified or a non-verified status), with or without explicitly using user templates. In the latter case, the comparands are implicitly implemented as part of the model. E.g., an ANN may not only comprise neural layers configured to extract features from the PPGs, but, in addition, include additional layers trained to perform the required inferences. In other variants, decision trees may be used in output of the feature extraction.

However, because of the time variability of the PPGs, it is advantageous to rely on user templates and occasionally update and rid the latter. Such comparands can easily be updated over time, whereas it is more difficult to retrain a cognitive model or re-parameterize a statistical model to adapt to evolving biometrics, particularly if such operations are carried out within an embedded solution, to ensure decentralization and privacy protection. Plus, using user templates requires less power consumption at runtime, as desired for embedded solutions.

As one understands, the time variability of user templates, often noted as a drawback in literature, can here advantageously be leveraged to increase security and privacy, inasmuch as the consequences of a lost or stolen user template are less dramatic than with face pictures and fingerprints, for example. So, a solution based on PPGs is less a concern for privacy and security. Still, a solution relying on user templates will typically need an initial enrolment, during which user templates are obtained and stored. To that aim, the present methods may further comprise initial steps of acquiring one or more initial user templates and storing the user templates in the device 10, e.g., during an enrolment phase. The enrolment phase typically uses the same feature extraction used by the matching procedure S40 but does not require to match the user.

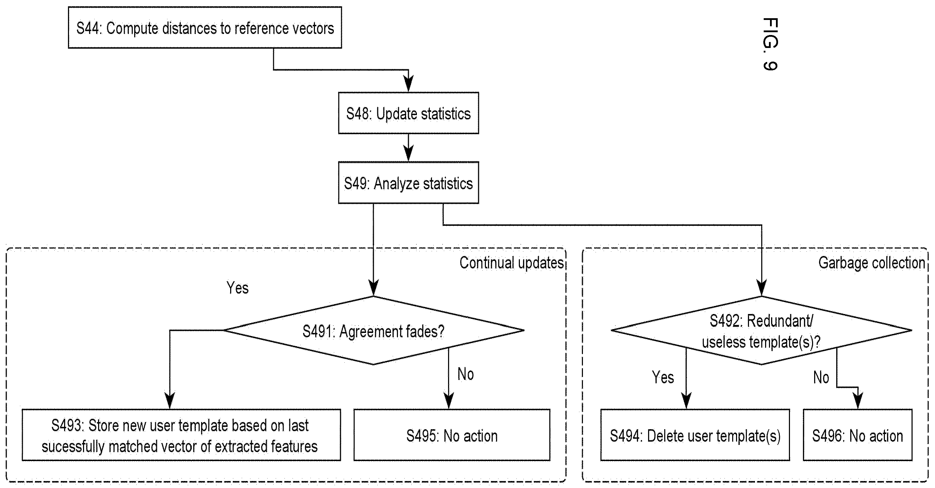

Updates to the user templates are now described in detail, in reference to FIG. 9. In embodiments, the user templates are continually updated S493, S494, whereby new user templates are stored S493 in the device 10. The new user templates are based on selected PPGs, which are successfully matched to the user at step S40. Meanwhile, user templates may have to be deleted S494 from the device 10, be it for memory reasons or because of the time variability issue noted above. Advantageously, the update procedure does not necessarily require user inputs, such as touching a device 10, staring at a screen, walking, typing a pattern, etc. The only constraint for the user may be to wear the device 10. On the contrary, a fingerprint reader requires the user to keep touching the sensor, while devices exploiting electrocardiogram (ECG) signals require to keep closing the electrical sensing circuit, e.g., with both hands touching the same device. On the contrary, embodiments disclosed herein allow the user to remain passive.

As further seen in FIG. 9, the present methods preferably update S48 statistics, continually, based on outcomes of the comparisons performed at step S44. In turn, user templates can be updated S493, S494 based on the updated statistics, e.g., thanks to simple analyses S49. Such statistics may notably include average distances to each of the current user templates, average minimal distance, average maximal distances, etc., match counts (counting how many times the templates are matched), and/or correlation metrics, as obtained upon comparing each successive test vector to the current user templates. Note, such statistics may possibly be all captured by a single value, obtained with an ad hoc metric, which is repeatedly updated. Also, the device 10 may possibly leverage different sets of templates associated with different user states. In this case, the statistics updates and analysis are performed in respect of relevant subsets of templates.

Updates S493, S494 to the user templates can for instance be decided based on updated statistics, by determining S491 whether to store a representation of a last successfully matched PPG (e.g., a corresponding test vector) as a new user template or not. In the affirmative (S491: Yes), a representation of the last successfully matched PPG (S45: Yes) is stored S493 as a new template in the device 10. Else (S491: No), no action is required. In variants, new user templates may be remotely obtained (e.g., via a server or smartphone) and then passed to the device 10. New user templates are typically stored when it is determined (S491: Yes) that the agreement with PPGs fades over time.

Embodiments may advantageously involve a garbage collection. That is, updates S493, S494 to the user templates may further involve the execution S492, S494, S496 of a garbage collection algorithm using the updated statistics as input, the aim being to delete S494 one or more user templates (as currently stored in the device 10) that become obsolete and/or redundant over time. As a general rule, this mechanism may rely on the frequency and precision with which the current templates are matched. I.e., user templates that are most frequently found to closely match the tested PPGs can be kept as reference templates, whereas templates that are rarely matched, or redundant, may be discarded. Of particular importance is to be able to remove redundant templates. So, the garbage collection mechanism may compare the current templates, based on the updated statistics, and only keep the most useful templates. Apart from data statistics, memory space may have to be taken into consideration as well.

In embodiments, the method leverages feature extraction, such that PPGs are captured as vectors and compared to user templates that are locally stored in the device 10, as reference vectors. This method is implemented at the biometric device 10 and comprises the following steps. PPGs are repeatedly obtained S37 by sensing S35 the user 1. At least some of these PPGs are being tested, for reasons explained earlier. For each tested PPG, features are extracted S43 as a test vector. Next, it is verified S44, S45 whether the test vector matches S45 a user template, by accessing one or more user templates and comparing S44 the test vector with the templates accessed. Interestingly, the user templates are stored in the biometric device 10 as reference vectors, which enables a quick verification and reduces the memory footprint of the templates. Such vectors are arrays of features that were previously extracted S43 from reference PPGs for the user 1. Thus, upon receiving a remote authentication request S70, the device may respond S90 to this request according to the outcome of the verification S44, S45.

As explained earlier, the comparison is preferably performed based on a distance metric, such as the Euclidean distance. As said, several user templates (or sets of templates) can be used, which may possibly depend on a current state of the user. Since the user templates are stored as vectors of extracted features on the device 10, their memory footprint is small and thus compatible with a user device 10 having limited memory capacity. Plus, the verification is easily and quickly done since a mere distance computation is needed for the comparison. Only the current PPG need be extracted as a feature vector; it is not needed to extract features from the stored user templates as the latter are already stored as extracted vectors. This makes it possible to verify the user locally more quickly (to authenticate the user at an external system 20 or 30), while requiring less memory.

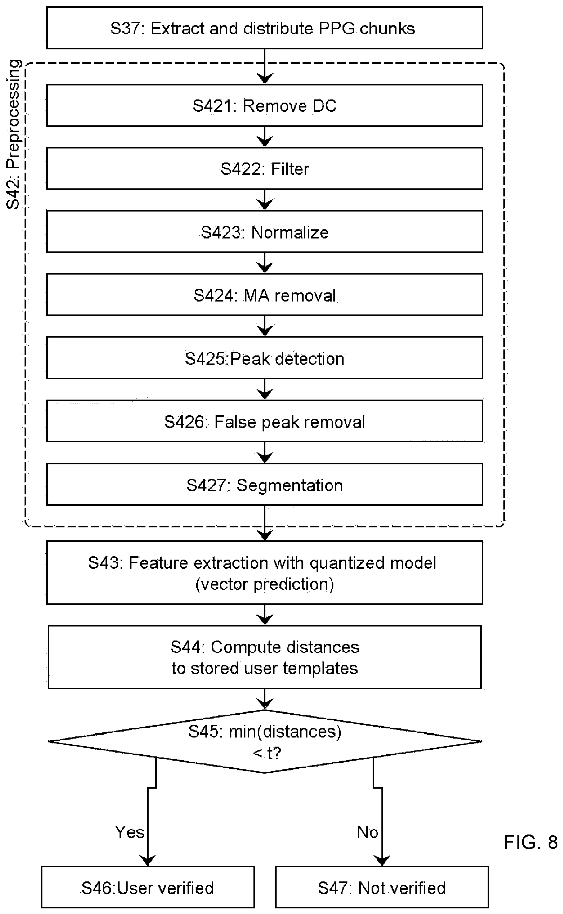

In embodiments, each PPG is obtained as a timeseries, i.e., as an object of the form $\{x_1, ..., x_{t-1}, x_t\}$, where the time information may be implicit (in particular if time intervals are constant). As explained earlier, the PPGs may be obtained from PPG signal, which is typically preprocessed, e.g., to filter noise, normalize and segment the signal. In particular, each PPG segment may be segmented to obtain S427 signal segments of a same reference length, as discussed in detail in section 2, in reference to FIG. 8.

Features are extracted S43 from PPGs (that may have a predefined length), using a pretrained extractor. The latter is implemented by an ANN, which preferably includes convolutional neural network (CNN) layers and/or recurrent neural network (RNN) layers. RNN layers help the model remembering the past and are thus well suited for handling timeseries. The RNN layers can for instance include stacked long short-term memory (LSTM) layers, as in preferred embodiments. More generally, the ANN may be configured as a temporal convolutional network. A preferred configuration of the ANN is discussed in section 2.

The extracted vectors are $m$-dimensional vectors, where, preferably, $m \geq 32$ (e.g., $m = 64$ or 128) and is typically less than or equal to 512 or 1024. The ANN extraction give rise to vectors having a small memory footprint, i.e., smaller than the PPG signal representation. The extraction can be constrained to make sure that all vectors are normalized to a same reference length, hence allowing more meaningful comparisons between the vectors.

The final classification is achieved based on the extracted vectors, e.g., by measuring distances (or correlations) with the reference vectors. A key advantage of using a pre-trained ANN as an extractor only is that a same extractor can be obtained, which is the same for all potential users. If the ANN would include additional layers for inferencing, then such layers would require a specific training for each user and specific training updates (i.e., new training steps) because of the time instability of PPGs, which would be dispiriting. Plus, such trainings and updates may have to be performed on a resource-constrained device (e.g., the biometric device 10 or a user computer 20), which may be difficult in practice. On the contrary, the proposed solution allows a same extractor to be trained (and possibly retrained) at a suitable computer and may leverage online mining and batching strategies. In variants, online mining and batching strategies may be used at an external device to train a subset of the layers of the ANN, which are later deployed at the device 10 (transfer learning). However, light training steps may be performed at the device 10 to train the residual layers responsible for inferencing.

Each vector can for instance be obtained in output of an L2 Normalization layer. Each vector is preferably a 128D vector, normalized using the L2 norm, and therefore pointing in a 128D hypersphere. Each of the vector component value is therefore less than or equal to 1. Other normalization schemes can be contemplated. Still, having vectors normalized all to a same length allows more meaningful comparisons.

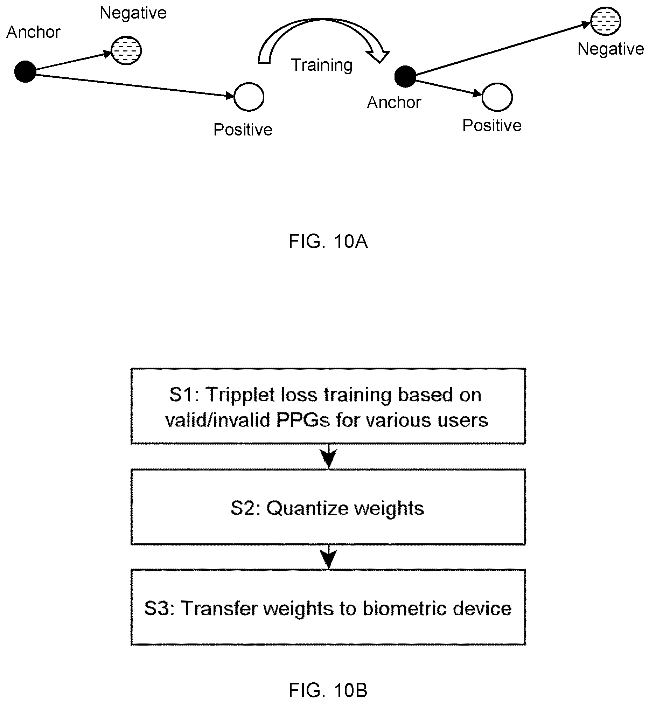

As noted above, the cognitive model is preferably trained off-device (i.e., on a remote computer), hence producing weights that are typically float32 or float64 numbers. The ANN architecture is meant to be deployed on a biometric device 10, which typically is a resource-constrained device 10. So, the ANN parameters are preferably quantized S2 (see FIG. 10B), according to an $m$-bit quantization scheme, where $m \leq 32$ and, preferably, $m = 8$. In practice, the ANN weights are typically quantized (before transferring S3 the model to the biometric device 10) from 32 to 8 bits, leading to integer values between -128 and 127. Quantization allows a more compact storage of the ANN parameters, a more efficient feature extraction process, and thus a more efficient verification S40 at the biometric device 10. Biases are typically nullified after quantization, which eventually reduces the number of required ANN parameters. In variants, though, the biometric device 10 may include a microchip supporting float32 ANN operations, in which case no quantization is strictly needed.

In embodiments, some feature engineering is involved prior to extracting features in the form of vectors. For example, continuous wavelet transforms (CWTs) of the PPG segments may be obtained. In variants, the extraction step S43 is based on signal features obtained from both the temporal space and its Fourier space (i.e., features in the frequency domain). In simpler variants, segments of the time-dependent signal are obtained, which correspond to an integer multiple of a quasi-period (corresponding to a cardiac cycle), and Fourier series coefficients $A_n$ are extracted from the segments, e.g., by locally fitting the Fourier series to a local segment. This amounts to virtually extrapolating the PPG segment to a periodic signal. A vector can thus be formed, based on the coefficients $A_n$ and the period of the Fourier series. The phase coefficient is excluded because it is useless. Doing so may drastically reduce the dimensionality of the problem, since only a few Fourier coefficients may be needed to fit the segment well. The vectors obtained may be directly used to compute distances. In variants, such vectors may be further processed through an ANN to extract further vectors, adequately normalized. The extractor may thus be leveraged to reduce or increase the dimensionality, if necessary.

Other types of extractors may be contemplated, which do not necessarily involve ANNs. For example, the features extracted may essentially be fiducial features. E.g., they may include a succession of time values corresponding to extrema of the PPG. In variants, each vector component involves a pair of values, i.e., including an extrema value and its corresponding time value. Such values can then be concatenated or composed as complex numbers to obtain vectors and then evaluate distances between the PPGs. Examples of fiducial features that can be exploited for matching PPG signals are the amplitudes of the extrema, their timing, in particular the systolic peak time and amplitude, the peak-peak interval, etc. Best results, however, have so far been obtained using an essentially non-fiducial approach (see the "peaks" algorithm described in section 2), by extracting features thanks to a pre-trained extractor configured as described above.

As illustrated in FIGS. 10A and 10B, the initial extractor is preferably trained S1 thanks to an $n$-uplet loss algorithm, where $n$ = 3 or 4. The $n$-uplet loss algorithm is trained according to a set of $n$-tuples. In addition to the anchor (corresponding to a given user), each tuple involves at least one representation of a valid PPG (i.e., belonging to the same given user) and at least one representation of an invalid PPG (e.g., belonging to a different user), as illustrated in FIG. 10A. E.g., a triplet-loss training algorithm is trained with triplets (3-tuples), each composed of an anchor, a positive, and a negative. Each 3-tuple is split into two pairs: anchor-positive and anchor-negative. Using a triplet loss algorithm proved to work surprisingly well. The same model can be used for all users based on a variety of valid and invalid examples. The trained cognitive model that results is thus agnostic to users. The trained model can thus be initially loaded, once for all, in biometric devices 10 (preferably after quantization), without requiring further individual parametrization by each user. Of particular advantage is that the trained model can be trained on a population of individuals because the model generalizes well to previously unseen individuals.

A triplet loss algorithm is used to ensure that a PPG $x_i^a$ (anchor) belonging to a specific individual is closer to all other PPGs $x_i^p$ (positive) belonging to the same individual than it is to any PPG $x_i^n$ (negative) belonging to any other individual. The triplet loss can be formulated as $L_{trp} = \sum_i^N \left[ \|f(x_i^a) - f(x_i^p)\|_2^2 - \|f(x_i^a) - f(x_i^n)\|_2^2 + \alpha_{trp} \right]_+$, where $[z]_+$ = max(z, 0), $f(x)$ is the extractor corresponding to extraction step S43, $\alpha_{trp}$ is an enforced margin between positive and negative pairs, and N is the cardinality of the set of all possible triplets in the training set. In embodiments, the Euclidean norm $\|x\|_2$ could be replaced by any other suitable distance metric $d(x)$.

The triplet loss algorithm trains the model based on the relative distances between positive and negative pairs with regards to the same PPG anchor (i.e., $x_i^a$). A quadruplet loss extends the triplet loss by introducing an additional constraint which pushes away negative pairs from positive pairs with regards to different anchor PPGs (i.e., $x_i^a$ and $x_i^l$). The quadruplet loss algorithm can be used to reduce intra-class variations and increase inter-class variations.

Note, additional signals may be acquired (such as IMU-related signals) to predict a current activity or state of the user and accordingly preselect user templates. In this case, user templates are sorted by user activity/state. Thus, the training of the extractor must be performed so as to take into account various possible states and activities of the users.

As noted earlier, each test vector can be compared S44 with the user templates using any suitable distance metric to obtain S44 distances, based on which it is verified S44, S45 whether the test vector matches S45 the user. The distance metric may for instance be a Euclidean distance. Some implementations may consider the minimum or the maximum of such distances. More generally, given a set of distances $\{d_1, \ldots, d_n\}$, an average distance may be computed according to a generalized mean formula, i.e., $M_p(d_1, \ldots, d_n) = \left( 1/n \sum_{i=1}^n d_i^p \right)^{1/p}$, where the parameter $p$ is set to an integer number, which determines the actual metric. The parameter $p$ shifts the generalized mean toward the maximum (positive $p$ values) or the minimum (negative $p$ values), which can be exploited to adapt the algorithm to the desired security level. Certain applications may require choosing a large parameter $p$, or even the max function, to provide better certainty (and increased security). I.e., if the maximal distance found is still under an acceptable threshold, the user 1 can safely be authenticated. On the contrary, other applications may rely on a negative parameter $p$, or the min function, to increase the chance to find a match (increased usability). In simpler variants, a test may be carried out based on both the min and max values. Various other heuristics can similarly be devised.

The user templates may possibly reside encrypted or obfuscated in the device 10. In that case, the templates have to be decrypted or de-obfuscated prior to verifying S40 the user. This way, the user templates cannot be easily extracted and stolen. For instance, the device may comprise a crypto security unit 180 configured to securely store and manage the templates. In variants, the user templates are stored obfuscated. E.g., the representations of the PPG segments are obfuscated (e.g., concatenated or interleaved) with elements of a secret key before the corresponding reference vectors are generated through the ANN; the resulting vectors can nevertheless be compared without de-obfuscation. This can notably be achieved with a modified triplet loss algorithm, where elements of the tuples are similarly obfuscated with keys, which do not need to be the same as the secret keys used in the biometric devices.

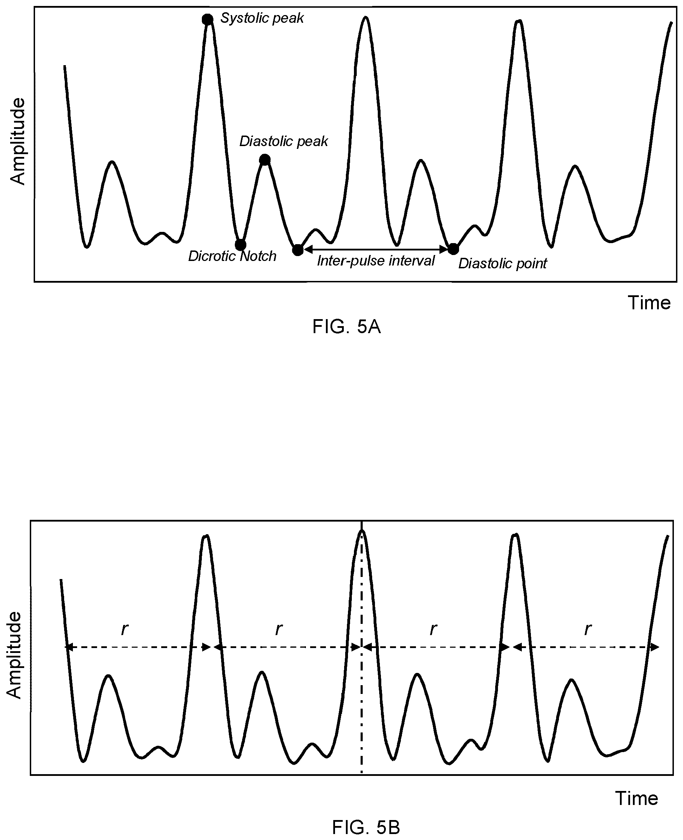

At runtime, each PPGs is preferably obtained S35, S36 by acquiring S35 a PPG signal, detecting S42 a systolic peak in the PPG signal acquired, and extracting a signal segment centred on the detected systolic peak, as illustrated in FIGS. 5A and 5B. As further seen in FIG. 5B, the signal segment extracted preferably has a predetermined length, equal to 4 $r$ in this example. That is, the algorithm takes two segments of length $r$ on each side of the central peak detected in the initial window. Such an approach is essentially non-fiducial and faster to execute, compared to a fiducial approach, which would require finding each peak, measuring amplitudes, time distances, etc. Preferably, several such segments are obtained and averaged to improve the signal-to-noise ratio (SNR).

The initial PPG signal is normally sampled to obtain a digital representation of the signal. The average sampling frequency may advantageously be between 5 and 7500 Hz, or preferably between 10 and 1000 Hz, or more preferably between 25 and 300 Hz. In particular, frequencies between 80 and 170 Hz turned out to be ideal in the present case. Such frequencies ensure that a sufficient amount of information is present in practice. Thus, less information is initially taken into account, compared with usual PPG sampling frequencies, which lowers the computational burden, but the information present is already sufficient to extract features that are relevant enough. Note, burst sampling can also be used, instead of uniform sampling; burst frequencies of more than 1 kHz may possibly be relied on.

The sampled signal is subsequently stored in the device, e.g., according to an $l$-bit resolution, where, e.g., $8 \leq l \leq 32$. The sampled signal values can for instance be stored at a low resolution, 8-bit, or 12-bit, to minimize the memory footprint, without substantially impacting the results.

Another aspect of the invention is now described in reference to FIGS. 1 - 4, which concerns a biometric system. The latter essentially comprises a biometric device 10, and may possibly include peripherals, such as a smartphone, a tablet, or a laptop 20 of the user, at which an access point is configured, as discussed earlier. Functional aspects of the device 10 have already been described in detail, in reference to the present methods. Such aspects are only briefly described in the following.

Essentially, the biometric device 10 comprises a sensing unit 100, which is configured to acquire PPG signals. It further includes an interface 160, which is designed to connect the device 10 to a computerized system 20, 30. The interface means may notably include a network interface, allowing the device 10 to communicate with a remote computerized system 30, e.g., via a network access point configured at a device 20. The interface means 160 may notably support Bluetooth, BLE, Universal Serial Bus (USB), and/or NFC connections.

The device further includes a processing unit 105 (e.g., a CPU) configured to take steps according to the present methods. To that aim, computerized methods may typically be stored in a permanent storage 150 of the device and loaded in the main memory 110 for execution by the CPU. In variants, such methods are hardcoded.

Preferably, the PPG frames are securely stored inside the device 10 only, e.g., in the storage 150. The local verification S40 takes place in the device 10. The processing unit 105 may possibly be configured to sample the PPG signals. In preferred variants, however, the digital conversion is ensured by an analogue-to-digital converter (ADC), which may possibly form part of the PPG sensor 104. Conversely, the PPG sensing mechanism typically include a light source 102, to which a digital-to-analogue converter (DAC) may possibly be coupled, as assumed in FIG. 3.

In simple embodiments, the user templates are stored in the clear, or are obfuscated or concealed. Preferably though, the verification S40 involves a key to decrypt the templates before comparison. To that aim, the device may include a crypto secure hardware element 180, enabling an entropy source. Note, the entropy source may possibly exploit the PPG signals themselves. The entropy source contribute to generate cryptographic objects such as asymmetric keys consumed by authentication protocols, such as involved in the FIDO2 set of specifications. The crypto secure hardware 180 can for instance be a crypto processor, e.g., as part of a system-on-chip (SoC) package. More generally, the device 10 can be designed to be compatible with external authentication protocols, to allow the user to be externally authenticated.

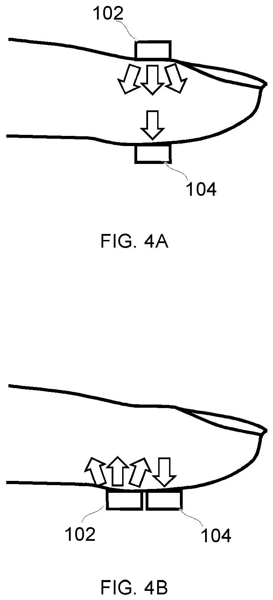

The sensing unit may notably include a PPG sensor 100, e.g., including a Light-Emitting Diode (LED) 102 and Photodiode (PD) 104 arranged in transmission (FIGS. 4A) or in reflection (FIG. 1A, 1B, 4B). For example, in reflexion, the sensor 100 may include a green light LED (with a wavelength at peak emission 515 nm) and a PD with peak sensitivity of 565 nm that measures light reflected by the skin. A green light works better in reflexion, while red light can penetrate deeper and thus work better in transmission. In variants, the PPG sensor 100 may involve one or more PDs and multiple LEDs, which may possibly use different wavelengths (e.g., green, red, infrared, etc.); the PDs may consistently be sensitive to multiple wavelengths.

The sensing unit optionally includes additional sensors, such as IMU sensors, proximity/capacity sensors, and/or an anti-forgery mechanism 170 (e.g., a mechanic ring protection mechanism or some sort of security circuit), as evoked earlier. In addition, the device may include an I/O interface 125 (for the user to switch on/off the device and interact with it), I/O controllers 120, one or more memory elements 110, 150, memory controllers 115, in addition to the processing means. A system bus 140 interfaces all components. In addition, the device 10 may include a battery (not shown) to power the device or be powered via an audio jack or a USB cable.

The device 10 typically includes one or more ADC and DAC converters, as peripherals on the chip, i.e., outside the CPU. As explained earlier, the device 10 may exploit interrupts, which can be implemented in hardware and/or in software. Preferably, all peripherals on the chip support interrupts. Interrupts are generated by events. Events can be generated by the peripherals themselves. A peripheral may generate multiple events with each event having a separate register in that peripheral's event register group. Peripherals can write and read events from and to registers without necessarily involving the CPU, to increase speed. For example: a hardware, real-time counter (RTC) peripheral may be used to generate interrupts to compare events every 0.01 seconds, which are then picked up by the ADC peripheral. This interrupt triggers an interrupt handler which reads a value from the ADC and stores it into a buffer. The ADC is connected to a PPG sensor 100; an int16 PPG value can for instance be obtained and stored in the buffer at every interrupt. Interrupts are typically prioritized. A possible priority order is the following (from highest priority to lowest): Acquiring PPG value > Assessing capacitive sensor > Acquiring user input through button1 > Acquiring user input through button2. Acquiring PPG signals has priority with respect to the sampling frequency (e.g., 100 Hz); assessing that the device is still being worn through the capacitive sensor also has high priority because it needs to be quick enough; finally, user buttons have lowest priority. Additional aspects of the device 10 are described in section 2.

Next, according to a final aspect, the invention can be embodied as a computer program product for authenticating a user with a biometric device 10. The computer program product comprises a computer readable storage medium having program instructions embodied therewith, where the program instructions are executable by the processing means 105 to cause the latter to take steps according to the present methods. Additional aspects of the computer program products are described in section 2.

The above embodiments have been succinctly described in reference to the accompanying drawings and may accommodate a number of variants. Several combinations of the above features may be contemplated. Examples are given in the next section.

## 2. Specific embodiments and technical implementation details

Embodiments of the proposed solution can be used to enable user authentication in various applications, e.g., digital signatures, user access to web sites, portals, and restricted areas. The proposed solution can be used to verify the physical identity of the user of a wearable biometric device 10, which functions like a passport to prove the user identity, e.g., online (to a sensible website like a bank) and/or offline (e.g., at an airport). The wearable device 10 can be paired to a companion device (e.g., a smartphone application) and/or a docking station for battery recharging, backups, and additional security measures. The proposed solution can further be used to monitor the digital presence and engagement of the user. In embodiments, the proposed solution offers various security enhancements, in terms of continuous user verification for remote authentication, continuous user template updates, and revocability of the user templates.

### 2.1 Preferred flows

### 2.1.1 High-level flow

A high-level flow of operations are shown in FIG. 6A. The biometric device is provided at step S10, the user enrols with the device and registers the device with local and/or remote computerized systems. At step S20, the user puts on the device, assumed to be a wearable device in this example. At S30, the user starts interacting with a smartphone, a tablet, or a computer. Meanwhile, the device repeatedly and continually updates S40, S50, S60 state parameters as described in the next subsection. When receiving S70 a request for remotely authenticating the user at one of said computerized systems, the device 10 checks S80 the current value of the causal state parameter and accordingly responds S90 to the remote request.

### 2.1.2 Preferred state parameter flows

State parameters can be handled as shown in FIG. 6B. At step S35, the biometric device senses the user to obtain PPG signal, which is stored S36 in a circular memory buffer. At step S37, the device extracts and distributes PPG signal chunks to each of the two procedures S40, S50. The first procedure S40 is the matching procedure, which starts by preprocessing S42 the chunks, see section 2.2. Features are then extracted S43 from the chunks to obtain a features vector. The corresponding distances to the reference vectors are computed at step S44. Step S45 checks whether a match is found. If so (S45: Yes), the first state parameter is set S46 to or maintained in its verified state, else it is set S47 to or maintained in its non-verified state. The user condition procedure S50 similarly includes pre-processing S52, albeit simpler than the pre-processing at step S42. Some signal features are identified at step S53 and analysed S54, to verify whether the signal reflects a HR, based on which the device assesses whether it is being worn S55. If yes, the first state parameter is set S56 to or maintained in a confirmed state. Else, it is set S57 to or maintained in a non-confirmed state. Such values may have to be logged unless the causal state parameter is immediately updated, as in preferred embodiments. A further sensor signal output (e.g., IMU) is collected at step S58, then processed S59 to verify whether the device was not removed. At step S60, the device updates the causal state parameter based on the current states of the first and second state parameters, so as to accordingly lock or unlock device.

Note, step S52 may, in variants, directly connect to S36 (and not S37), as suggested by the dashed arrow, so as to process more data. A similar result may also be obtained by storing statistics of the features analysis S54 to be used across loops of the procedure S50. Both strategies are especially useful where HR monitoring is used to check the user condition.

The causal state parameter is preferably updated S60 immediately, e.g., thanks to simple Boolean comparisons. These may possibly be triggered by interrupts or exit conditions in loops, as explained earlier. If the last PPG was successfully matched (S45: Yes), the output of S45 is TRUE, else it is FALSE. Similarly, if the user condition was confirmed to remain stable (S55: Yes), the output of S55 is TRUE, else it is FALSE. Updating any of the state parameters in output of steps S45 and S55 triggers an AND comparison. The latter requires both state parameters to be TRUE to set or maintain the causal state parameter to unlocked, else the device is locked. Note, such steps can equivalently be handled by way of any binary values (e.g., "0" and "1"), as explained in section 1.

### 2.1.3. Updates and garbage collection

FIG. 9 exemplifies a mechanism for managing updates and garbage collection. Steps S44 and S45 have been described above. Statistics can be updated based on outputs of each of steps S44 (e.g., to update average distances and/or correlations) and S45 (to update counts). Such statistics are analysed at step S49, so as to continually update the user templates or delete them. If it turns out that some of the user templates (i.e., reference vectors in this example) become useless over time (e.g., the agreement fades, S491: Yes), then the method instructs S493 to store S493 a fresh template, based on the last test vector that was found to successfully match the user. Else, if the user templates are still valid (S491: No), no specific action is required.

The garbage collection mechanism checks S492 whether the user templates become redundant (i.e., some templates happen to be matched, but have no added value with respect to other stored templates) or otherwise useless (because they are never or infrequently matched). If so, the corresponding user templates can be deleted S494, else no action is required S496. The available memory may further be checked, which may trigger template deletion too (not shown).

### 2.1.4 Preferred state diagram

A state diagram is shown in FIG. 7, according to which the device 10 is in one of two possible states ("locked" and "unlocked"), in accordance with states of the causal state parameters and other factors. Basically, when being in the locked state, the device 10 remains in the locked state during and after obtaining a new PPG, during the matching procedure, and if the matching procedure fails. The user may possibly be directly notified via a display or an LED on the device 10. Conversely, the device may be unlocked if the PPG is successfully matched (the user may accordingly be notified). The device will remain unlocked while obtaining a new PPG, performing a further matching procedure, or if the further PPG is successfully matched. Finally, the device may be set to the locked state if the further PPG is not matched to the user, if the device is removed (or forced), or if a timer expires before the next successful PPG match. Note, if the device is switched off, it is in a state (off) that is equivalent to the locked state as the device 10 cannot positively respond to a remote request S70 any longer.

### 2.2 Signal pre-processing

PPG chunks are extracted and distributed at step S37. Such "chunks" are initial (rough) segments of the PPG signal. Pre-processing is performed at step S42, which is described below in detail. This step results in PPG segments, from which features are extracted as vectors, on-device, using the quantized ANN, at step S43. Distances to the reference vectors are computed at step S44. Next, the method checks whether the minimum of all the obtained distances is strictly less than a given threshold $t$ at step S45.

A preferred pre-processing pipeline S42 is the following. The first step S421 is to remove the 0 Hz component, also called DC bias. The following step S422 filters the obtained signal. E.g., for this step a Butterworth bandpass filter of order 4 and cut-off frequencies of 0.5 Hz and 5 Hz can be used. The resulting signal is then normalized S423, based on minimal and maximal values of the filtered signal. Next, motion artefacts (MAs) are removed at step S424. IMU signals can for instance be used to identify at which frequencies MAs occur in order to subtract them from the PPG, or in order to fully discard the PPG signal when disturbed by motion. Step S425 concerns the systolic peak detection, which is employed if the segmentation technique adopted is "peaks" or "raw peaks", see below. False peaks are removed at step S426, if necessary. A simple removal technique is based on the peak height and prominence, and the minimum distance between the peaks. A further segmentation is performed at step S427, to split the signal (or its chunks) into smaller units that will form the model's input features.

Three different segmentation techniques were tested. A first segmentation is based on time: the input is simply split into segments of defined duration. When this segmentation technique is used, it is useless to detect peaks and remove false peaks. In a variant called "peaks", an input chunk is split around a detected systolic peaks by selecting an arbitrary number $p$ of signal periods, as assumed in FIG. 5B, where $p = 4$. The length $r$ of each period is set according to the average HR of the user and is retrieved during the pre-processing. As users typically have different HRs, the segment durations will typically differ from one user to the other. Still, the segments can be padded (adding zeros) to some predefined segment length (e.g., an estimate of the longest typical segment duration for a given value of $p$) before being processed by the ANN.

Alternatives to padding are interpolation or extrapolation. Another possibility, called "raw peaks", is to split the input in consecutive segments composed of a given number $p$ of peaks. This time the segments are generated without using the average HR. However, as the present inventor concluded, the "peaks" approach works better than the others as it allows users to be better distinguished.

## 2.3 Preferred ANN architecture

The following describes a preferred model architecture used to train the extraction model. The aim is to learn a Euclidean embedding per input feature vector by using a deep convolutional network. The network is trained such that the squared L2 distances in the embedding space directly correspond to segment similarity: segments of the same person have small distances and segments of distinct people have large distances. Once an embedding has been produced from an input PPG signal segment, the person verification simply amounts to thresholding the distances to embeddings corresponding to user templates. The preferred architecture is summarized in Table I.

Table I: Preferred ANN architecture

| Layer (type) | Output Shape | Param # |
|---|---|---|
| Input (InputLayer) | [(None, 600, 1)] | 0 |
| Conv1D 1 (Conv1D) | (None, 571, 32) | 992 |
| MaxPooling1D 1 (MaxPooling1D) | (None, 142, 32) | 0 |
| Dropout 1 (Dropout) | (None, 142, 32) | 0 |
| Conv1D 2 (Conv1D) | (None, 93, 32) | 51232 |
| MaxPooling1D 2 (MaxPooling1D) | (None, 23, 32) | 0 |
| Dropout 2 (Dropout) | (None, 23, 32) | 0 |
| LSTM 1 (LSTM) | (None, 23, 128) | 82432 |
| LSTM 2 (LSTM) | (None, 128) | 131584 |
| L2Norm (L2 Normalization) | (None, 128) | 0 |

The input layer picks up signal segment values. The Conv1D layers present strides equal to 1, no padding, and are initialized with the Glorot uniform initializer, also called Xavier uniform initializer. The MaxPool1D layers present maximum pooling window of 4 and no padding. When the activation layer is a Rectified Linear Unit (ReLU), the Dropout layers present a dropout rate of 0.1. If a Scaled Exponential Linear Unit (SELU) is used instead, then AlphaDropout layers with dropout rate of 0.1 can be used in place of the Dropout layers. The LSTM layers employ Sigmoid activation function for the recurrent step, Glorot uniform kernel initializer, and an orthogonal initializer for the linear transformation of the recurrent state. The result is a vector with length 128 which is normalized according to L2 norm to produce embeddings that are then used for both training (at build time) and user verification (at run time).

## 2.4. Final considerations

Computerized devices can be suitably designed for implementing embodiments of the present invention as described herein. In that respect, it can be appreciated that the methods described herein are at least partly non-interactive, i.e., automated. Automated parts of such methods can be implemented in software, hardware, or a combination thereof. In exemplary embodiments, automated parts of the methods described herein are implemented in software, as a service or an executable program (e.g., an application), the latter executed by suitable digital processing devices. Aspects of the present invention are described herein notably with reference to flowcharts and block diagrams. It will be understood that each block, or combinations of blocks, of the flowchart and the block diagram can be implemented by computer readable program instructions. The flowchart and the block diagram in the accompanying drawings illustrate the architecture, functionality, and operation of possible implementations of the biometric device 10, methods of operating it, and computer program products according to various embodiments of the present invention. Note that each computer-implemented block in the flowchart or the block diagram may represent a module, or a portion of instructions, which comprises executable instructions for implementing the functions or acts specified therein. In variants, the functions or acts mentioned in the blocks may occur out of the order specified in the figures. For example, two blocks shown in succession may actually be executed in parallel, concurrently, or still in a reverse order, depending on the functions involved and the algorithm optimization retained. It is also reminded that each block and combinations thereof can be adequately distributed among special purpose hardware components.

While the present invention has been described with reference to a limited number of embodiments, variants, and the accompanying drawings, it will be understood by those skilled in the art that various changes may be made, and equivalents may be substituted without departing from the scope of the present invention as defined in the accompanying claims. In particular, a feature (device-like or method-like) recited in a given embodiment, variant, aspect, or shown in a drawing may be combined with or replace another feature in another embodiment, variant, aspect, or drawing, without departing from the scope of the present invention as defined in the accompanying claims. Various combinations of the features described in respect of any of the above embodiments or variants may accordingly be contemplated, that remain within the scope of the appended claims. In addition, many minor modifications may be made to adapt a particular situation or material to the teachings of the present invention without departing from its scope, as defined in the accompanying claims. Therefore, it is intended that the present invention not be limited to the particular embodiments disclosed, but that the present invention will include all embodiments falling within the scope of the appended claims. In addition, many other variants than explicitly touched above can be contemplated.

**Claims**

1. A method of authenticating a user with a biometric device (10), the method comprising, at the biometric device (10):

   repeatedly sensing (S35) the user to obtain (S37) photoplethysmograms, or PPGs, and verifying whether at least some of the PPGs obtained match (S45) the user or not, by executing (S40) a matching procedure;
   repeatedly determining (S50, S55), based on sensor measurements, whether a condition of the user remains stable or not;
   repeatedly updating (S60) a causal state parameter to set it to an unlocked state only if a last verified PPG of said PPGs matches the user and the condition of the user is determined to have remained stable since a time at which the last verified PPG matched the user, the causal state parameter being else set to a locked state, and
   upon receiving (S70) a request originating from a computerized system (20, 30) to authenticate the user (1), checking (S80) a current state of the causal state parameter, and responding (S90) to the request to allow the user to be authenticated only if the current state of the causal state parameter is in its unlocked state,
   wherein
   the user is repeatedly sensed (S35) to obtain (S36) a PPG signal, and
   the method is **characterized by** extracting (S37)

      first portions of the PPG signal to form said PPGs and verifying whether said at least some of the PPGs obtained match (S45) the user, and
      second portions of the PPG signal, based on which it is determined (S52 - S55) whether said condition remains stable or not.

2. The method according to claim **1,** wherein

   the biometric device (10) is a wearable device,
   determining (S50, S55) whether said condition remains stable or not includes determining (S50) whether the biometric device (10) is worn by the user (1), by executing (S52 - S55) a confirmation procedure using said second portions as input, wherein the confirmation procedure differs from the matching procedure executed to verify (S42 - S45) whether said at least some of the PPGs match the user, and
   the computer system (30) is preferably a remote computerized system, to which the wearable device more preferably connects via an access point (20).

3. The method according to claim **2,** wherein
   the confirmation procedure is executed at an average frequency that is higher than an average frequency at which the matching procedure is executed.

4. The method according to any one of claims 1 to 3, wherein the method further comprises
   repeatedly updating two further state parameters, including a first state parameter and a second state parameter, wherein

      the first state parameter is set (S46, S47) to a verified state (S46) or a non-verified state (S47), depending on whether the last verified PPG matches the user or not, respectively;

the second state parameter is set (S56, S57) to a confirmed state (S56) or a non-confirmed state (S57), depending on whether the condition of the user and/or the device is determined to remain stable or not, respectively; and

at repeatedly updating (S60) the causal state parameter, the causal state parameter is set to the unlocked state only if the first state parameter is currently in the verified state and the second state parameter was always in the confirmed state since the time at which the last verified PPG matched the user, the causal state parameter being else set to a locked state.

5. The method according to claim **4,** wherein
the causal state parameter is immediately updated (S60) upon updating (S46, S47, S56, S57) any of the first state parameter and the second state parameter, so as to be set (S60) to the locked state as soon as the first state parameter is set to the non-verified state or the second state parameter is set to the non-confirmed state.

6. The method according to any one of claims **1** to **5,** wherein
the method further comprises continually storing (S36) values representing the sensed PPG signal in a circular memory buffer, the latter designed so as to store a finite time duration of the PPG signal sensed, and the PPGs are obtained (S37) based on the stored values.

7. The method according to any one of claims **1** to **6,** wherein
verifying (S40) whether said at least some of the PPGs obtained match the user (1) comprises, for each PPG of said at least some of the PPGs obtained, accessing (S44) one or more user templates stored on the biometric device (10) and comparing (S44) said each PPG to each of the one or more user templates accessed, to verify whether said each PPG matches (S45) any one of the one or more user templates.

8. The method according to claim 7, wherein

verifying (S40) whether said each PPG matches the user (1) further comprises extracting (S43) features of said each PPG as a test vector,
the one or more user (1) templates are stored on the biometric device (10) as one or more reference vectors, respectively, wherein the one or more reference vectors were previously obtained as features extracted from one or more reference PPGs for this user (1), and
comparing (S44) said each PPG to each of the one or more user templates accessed comprises comparing (S44) the test vector with the one or more reference vectors, preferably by computing distances between, on the one hand, the test vector and, on the other hand, the one or more reference vectors.

9. The method according to any one of claims **1** to **8,** wherein
the method further comprises continually updating (S493, S494) the one or more user (1) templates, whereby

new user templates are stored (S493) in the device (10), the new user templates based on selected ones of the matched PPGs, and,
preferably, selected user templates are deleted (S494) from the device (10).

10. The method according to claim **9,** wherein

the one or more user templates include several user templates,
the method further comprises updating (S48) statistics based on an outcome of comparing said each PPG to each of the several user templates,
the user templates are updated (S493, S494) based (S49) on the updated statistics.

11. The method according to claim **10,** wherein
updating (S493, S494) the user templates comprises determining (S491), based (S49) on the updated statistics, whether to store a representation of said each PPG as a new user template or not, and, in the affirmative, storing (S493) this representation as a new template in the device (10).

12. The method according to any one of claims **9** to **11,** wherein
updating (S493, S494) the user templates comprises executing (S492, S494, S496) a garbage collection algorithm using (S49) the updated statistics as input, to delete (S494) one or more of the user templates as currently stored in the device (10).

13. The method according to any one of claims **1** to **12,** wherein

the computerized system is a remote computerized system, and
the request is received (S70) and responded (S90) to in accordance with one or more authentication protocols of a logical authentication specification and/or a physical authentication specification.

14. A biometric system comprising a biometric device (10), where the biometric device comprises

a sensing unit (100) configured to acquire photoplethysmogram signals;
an interface (160) configured to connect the biometric device (10) to a computerized system (20, 30), preferably a remote computerized system (30), more preferably via a network access point (20);
a processing unit (105) configured to take steps according to the method of any one of claims **1** to 13.

15. A computer program product for authenticating a user (1) with a biometric device (10), the computer program product comprising a computer readable storage medium having program instructions embodied therewith, the program instructions executable by processing means of the biometric device to cause the latter to take steps according to the method of any one of claims **1** to 13.


**Patentansprüche**

1. Ein Verfahren zur Authentifizierung eines Benutzers mit einem biometrischen Gerät (10), wobei das Verfahren am biometrischen Gerät (10) umfasst:

wiederholtes Erfassen (S35) des Benutzers, um Photoplethysmogramme oder PPGs zu erhalten (S37), und Überprüfen, ob mindestens einige der erhaltenen PPGs mit dem Benutzer übereinstimmen (S45) oder nicht, durch Ausführen (S40) eines Abgleichverfahrens;
wiederholtes Bestimmen (S50, S55) auf der Grundlage von Sensormessungen, ob ein Zustand des Benutzers stabil bleibt oder nicht;
wiederholtes Aktualisieren (S60) eines kausalen Zustandsparameters, um ihn nur dann in einen entsperrten Zustand zu versetzen, wenn ein zuletzt überprüftes PPG der PPGs mit dem Benutzer übereinstimmt und festgestellt wird, dass der Zustand des Benutzers seit dem Zeitpunkt, zu dem das zuletzt überprüfte PPG mit dem Benutzer übereinstimmte, stabil geblieben ist, wobei der kausale Zustandsparameter andernfalls in einen gesperrten Zustand versetzt wird, und
nach Empfang (S70) einer Anfrage, die von einem computergestützten System (20, 30) zur Authentifizierung des Benutzers (1) stammt, Überprüfen (S80) eines aktuellen Zustands des kausalen Zustandsparameters und Antworten (S90) auf die Anfrage, um dem Benutzer die Authentifizierung nur dann zu ermöglichen, wenn sich der aktuelle Zustand des kausalen Zustandsparameters in seinem entsperrten Zustand befindet,
wobei der Benutzer wiederholt erfasst wird (S35), um ein PPG-Signal zu erhalten (S36), und
das Verfahren durch das Extrahieren (S37) erster Teile des PPG-Signals, um die PPGs zu bilden und das Überprüfen, ob mindestens einige der erhaltenen PPGs mit dem Benutzer übereinstimmen (S45), und
zweiter Teile des PPG-Signals, auf deren Grundlage bestimmt wird (S52 - S55), ob der Zustand stabil bleibt oder nicht,
gekennzeichnet ist.

2. Das Verfahren nach Anspruch **1,** wobei

die biometrische Vorrichtung (10) eine tragbare Vorrichtung ist,
Bestimmen (S50, S55), ob der Zustand stabil bleibt oder nicht, das Bestimmen (S50) umfasst, ob die biometrische Vorrichtung (10) vom Benutzer (1) getragen wird, indem (S52 - S55) ein Bestätigungsverfahren unter Verwendung der zweiten Teile als Eingabe ausgeführt wird, wobei sich das Bestätigungsverfahren vom Abgleichverfahren unterscheidet, das ausgeführt wird, um zu überprüfen (S42 - S45), ob mindestens einige der PPGs mit dem Benutzer übereinstimmen, und
das Computersystem (30) vorzugsweise ein entferntes Computersystem ist, mit dem das tragbare Gerät vorzugsweise über einen Zugangspunkt (20) verbunden ist.

3. Das Verfahren nach Anspruch **2,** wobei
das Bestätigungsverfahren mit einer durchschnittlichen Frequenz ausgeführt wird, die höher ist als die durchschnittliche Frequenz, mit der das Abgleichverfahren ausgeführt wird.

4.  Das Verfahren nach einem der Ansprüche **1** bis **3,** wobei das Verfahren weiter umfasst

    wiederholtes Aktualisieren von zwei weiteren Zustandsparametern, einschliesslich eines ersten Zustandsparameters und eines zweiten Zustandsparameters, wobei
    der erste Zustandsparameter (S46, S47) auf einen verifizierten Zustand (S46) oder einen nicht verifizierten Zustand (S47) gesetzt wird, je nachdem, ob das zuletzt verifizierte PPG mit dem Benutzer übereinstimmt oder nicht;
    der zweite Zustandsparameter (S56, S57) auf einen bestätigten Zustand (S56) oder einen nicht bestätigten Zustand (S57) gesetzt wird, je nachdem, ob der Zustand des Benutzers und/oder des Geräts als stabil oder nicht stabil ermittelt wurde; und
    bei wiederholter Aktualisierung (S60) des kausalen Zustandsparameters wird der kausale Zustandsparameter nur dann auf den entsperrten Zustand gesetzt, wenn sich der erste Zustandsparameter derzeit im verifizierten Zustand befindet und sich der zweite Zustandsparameter seit dem Zeitpunkt, zu dem die letzte verifizierte PPG mit dem Benutzer übereinstimmte, immer im bestätigten Zustand befand, wobei der kausale Zustandsparameter andernfalls auf einen gesperrten Zustand gesetzt wird.

5.  Das Verfahren nach Anspruch **4,** wobei
    der kausale Zustandsparameter sofort aktualisiert wird (S60), wenn der erste Zustandsparameter oder der zweite Zustandsparameter aktualisiert wird (S46, S47, S56, S57), so dass er (S60) auf den gesperrten Zustand gesetzt wird, sobald der erste Zustandsparameter auf den nicht verifizierten Zustand oder der zweite Zustandsparameter auf den nicht bestätigten Zustand gesetzt wird.

6.  Das Verfahren nach einem der Ansprüche **1** bis **5,** wobei
    das Verfahren weiter das kontinuierliche Speichern (S36) von Werten umfasst, die das erfasste PPG-Signal in einem zirkulären Speicherpuffer darstellen, wobei letzterer so ausgelegt ist, dass er eine endliche Zeitdauer des erfassten PPG-Signals speichert, und die PPGs auf der Grundlage der gespeicherten Werte erhalten werden (S37).

7.  Das Verfahren nach einem der Ansprüche **1** bis **6,** wobei
    das Überprüfen (S40), ob die mindestens einigen der erhaltenen PPGs mit dem Benutzer (1) übereinstimmen, für jedes PPG der mindestens einigen erhaltenen PPGs das Zugreifen (S44) auf eine oder mehrere auf dem biometrischen Gerät (10) gespeicherte Benutzervorlagen und das Vergleichen (S44) jedes PPG mit jeder der einen oder mehreren Benutzervorlagen, umfasst, um zu überprüfen, ob jedes PPG mit einer der einen oder mehreren Benutzervorlagen übereinstimmt (S45).

8.  Das Verfahren nach Anspruch **7,** wobei

    das Überprüfen (S40), ob jedes PPG mit dem Benutzer (1) übereinstimmt, weiter das Extrahieren (S43) von Merkmalen jedes PPG als Testvektor umfasst,
    die eine oder mehreren Benutzervorlagen (1) auf dem biometrischen Gerät (10) als einen oder mehrere Referenzvektoren gespeichert sind, wobei der eine oder die mehreren Referenzvektoren zuvor als Merkmale extrahiert wurden, die aus einem oder mehreren Referenz-PPGs für diesen Benutzer (1) extrahiert wurden, und Vergleichen (S44) jedes PPG mit jeder der einen oder mehreren Benutzervorlagen, auf die zugegriffen wurde, das Vergleichen (S44) des Testvektors mit dem einen oder den mehreren Referenzvektoren umfasst, vorzugsweise durch Berechnen von Abständen zwischen einerseits dem Testvektor und andererseits dem einen oder den mehreren Referenzvektoren.

9.  Das Verfahren nach einem der Ansprüche **1** bis **8,** wobei

    das Verfahren weiter das kontinuierliche Aktualisieren (S493, S494) der einen oder mehreren Benutzervorlagen (1) umfasst, wobei
    neue Benutzervorlagen in der Vorrichtung (10) gespeichert werden (S493), wobei die neuen Benutzervorlagen auf ausgewählten der übereinstimmenden PPGs basieren, und
    vorzugsweise ausgewählte Benutzervorlagen aus der Vorrichtung (10) gelöscht werden (S494).

10. Das Verfahren gemäß Anspruch **9,** wobei

die eine oder mehreren Benutzervorlagen mehrere Benutzervorlagen umfassen,

das Verfahren weiter das Aktualisieren (S48) von Statistiken auf der Grundlage eines Ergebnisses des Vergleichs jedes PPG mit jeder der mehreren Benutzervorlagen umfasst,

die Benutzervorlagen auf der Grundlage (S49) der aktualisierten Statistiken aktualisiert werden (S493, S494).

11. Das Verfahren nach Anspruch **10,** wobei

das Aktualisieren (S493, S494) der Benutzervorlagen das Bestimmen (S491) auf der Grundlage (S49) der aktualisierten Statistiken, ob eine Darstellung jedes PPG als neue Benutzervorlage gespeichert werden soll oder nicht, und, falls ja, das Speichern (S493) dieser Darstellung als neue Vorlage in dem Gerät (10), umfasst.

12. Das Verfahren nach einem der Ansprüche **9** bis **11,** wobei

das Aktualisieren (S493, S494) der Benutzervorlagen das Ausführen (S492, S494, S496) eines Garbage-Collection-Algorithmus unter Verwendung (S49) der aktualisierten Statistiken als Eingabe umfasst, um eine oder mehrere der derzeit in der Vorrichtung (10) gespeicherten Benutzervorlagen zu löschen (S494).

13. Das Verfahren nach einem der Ansprüche **1** bis **12,** wobei

das computergestützte System ein ferngesteuertes computergestütztes System ist und

die Anfrage gemäss einem oder mehreren Authentifizierungsprotokollen einer logischen Authentifizierungsspezifikation und/oder einer physikalischen Authentifizierungsspezifikation empfangen (S70) und beantwortet (S90) wird.

14. Ein biometrisches System, das eine biometrische Vorrichtung (10) umfasst, wobei die biometrische Vorrichtung umfasst

eine Erfassungseinheit (100), die zum Erfassen von Photoplethysmogrammsignalen konfiguriert ist;

eine Schnittstelle (160), die so konfiguriert ist, dass sie das biometrische Gerät (10) mit einem Computersystem (20, 30), vorzugsweise einem entfernten Computersystem (30), vorzugsweise über einen Netzwerkzugangspunkt (20), verbindet;

eine Verarbeitungseinheit (105), die so konfiguriert ist, dass sie Schritte gemäss dem Verfahren nach einem der Ansprüche **1** bis **13** ausführt.

15. Ein Computerprogrammprodukt zur Authentifizierung eines Benutzers (1) mit einem biometrischen Gerät (10), wobei das Computerprogrammprodukt ein computerlesbares Speichermedium mit darauf gespeicherten Programmbefehlen umfasst, wobei die Programmbefehle durch eine Verarbeitungseinrichtung des biometrischen Geräts ausgeführt werden können, um dieses zu veranlassen, Schritte gemäss dem Verfahren nach einem der Ansprüche **1** bis **13** durchzuführen.

## Revendications

1. Procédé d'authentification d'un utilisateur à l'aide d'un dispositif biométrique (10), le procédé comprenant, au niveau du dispositif biométrique (10) :

détecter de manière répétée (S35) l'utilisateur afin d'obtenir (S37) des photopléthysmogrammes, ou PPG, et vérifier si au moins certains des PPG obtenus correspondent (S45) à l'utilisateur ou non, en exécutant (S40) une procédure de correspondance ;

déterminer de manière répétée (S50, S55), sur la base des mesures du capteur, si la condition de l'utilisateur reste stable ou non ;

mettre à jour de manière répétée (S60) un paramètre d'état causal pour le régler sur un état déverrouillé uniquement si un dernier PPG vérifié parmi lesdits PPG correspond à l'utilisateur et s'il est déterminé que la condition de l'utilisateur est restée stable depuis un temps auquel le dernier PPG vérifié correspondait à l'utilisateur, le paramètre d'état causal étant sinon réglé sur un état verrouillé, et

à réception (S70) d'une demande provenant d'un système informatisé (20, 30) pour authentifier l'utilisateur (1), vérifier (S80) un état actuel du paramètre d'état causal, et répondre (S90) à la demande pour permettre à l'utilisateur d'être authentifié uniquement si l'état actuel du paramètre d'état causal est dans son état déverrouillé,

dans lequel :

l'utilisateur est détecté de manière répétée (S35) afin d'obtenir (S36) un signal PPG, et
le procédé est **caractérisé en ce qu'**il comprend en outre l'extraction (S37)

de premières parties du signal PPG pour former lesdits PPG pour vérifier si au moins certains des PPG obtenus correspondent (S45) à l'utilisateur, et
de secondes parties du signal PPG, sur la base desquelles il est déterminé (S52 - 555) si ladite condition reste stable ou non.

2. Procédé selon la revendication **1,** dans lequel

le dispositif biométrique (10) est un dispositif portable,
déterminer (S50, S55) si ladite condition reste stable ou non comprend déterminer (S50) si le dispositif biométrique (10) est porté par l'utilisateur (1), en exécutant (S52 - 555) une procédure de confirmation utilisant lesdites secondes parties comme entrée, dans laquelle la procédure de confirmation diffère de la procédure de correspondance exécutée pour vérifier (S42 - S45) si au moins certaines des PPG correspondent à l'utilisateur, et
le système informatisé (30) est de préférence un système informatisé distant, auquel le dispositif portable se connecte plus préférablement via un point d'accès (20).

3. Procédé selon la revendication **2,** dans lequel
la procédure de confirmation est exécutée à une fréquence moyenne supérieure à la fréquence moyenne à laquelle la procédure de correspondance est exécutée.

4. Procédé selon l'une quelconque des revendications **1** à **3,** dans lequel le procédé comprend en outre

la mise à jour répétée de deux autres paramètres d'état, comprenant un premier paramètre d'état et un deuxième paramètre d'état, dans lequel
le premier paramètre d'état est défini (S46, S47) sur un état vérifié (S46) ou un état non vérifié (S47), selon que le dernier PPG vérifié correspond ou non à l'utilisateur, respectivement ;
le deuxième paramètre d'état est défini (S56, 557) sur un état confirmé (S56) ou un état non confirmé (S57), selon qu'il est déterminé que la condition de l'utilisateur et/ou du dispositif reste stable ou non, respectivement ; et
lors de la mise à jour répétée (S60) du paramètre d'état causal, le paramètre d'état causal est défini sur l'état déverrouillé uniquement si le premier paramètre d'état est actuellement dans l'état vérifié et si le deuxième paramètre d'état était toujours dans l'état confirmé depuis un temps auquel le dernier PPG vérifié correspondait à l'utilisateur, le paramètre d'état causal étant sinon défini sur un état verrouillé.

5. Procédé selon la revendication **4,** dans lequel
le paramètre d'état causal est immédiatement mis à jour (S60) lors de la mise à jour (S46, S47, S56, S57) de l'un quelconque des premier et deuxième paramètres d'état, de manière à être défini (S60) sur l'état verrouillé dès que le premier paramètre d'état est défini sur l'état non vérifié ou que le deuxième paramètre d'état est défini sur l'état non confirmé.

6. Procédé selon l'une quelconque des revendications **1** à **5,** dans lequel
le procédé comprend en outre le stockage continu (S36) de valeurs représentant le signal PPG détecté dans une mémoire tampon circulaire, cette dernière étant conçue pour stocker une durée finie du signal PPG détecté, et les PPG sont obtenus (S37) sur la base des valeurs stockées.

7. Procédé selon l'une quelconque des revendications **1** à **6,** dans lequel
vérifier (S40) si au moins certains des PPG obtenues correspondent à l'utilisateur (1) comprend, pour chacun desdits PPG obtenus, accéder (S44) à un ou plusieurs modèles d'utilisateurs stockés sur le dispositif biométrique (10) et comparer (S44) chaque PPG à chacun des un ou plusieurs modèles d'utilisateurs auxquels on a accédé, afin de vérifier si chaque PPG correspond (S45) à l'un quelconque des un ou plusieurs modèles d'utilisateurs.

8. Procédé selon la revendication **7,** dans lequel

vérifier (S40) si chaque PPG correspond à l'utilisateur (1) comprend en outre l'extraction (S43) des caractéristiques de chaque PPG en tant que vecteur de test,
le ou les modèles d'utilisateur (1) sont stockés sur le dispositif biométrique (10) sous la forme d'un ou plusieurs vecteurs de référence, respectivement, dans lequel le ou les vecteurs de référence ont été préalablement obtenus en tant que caractéristiques extraites d'un ou plusieurs PPG de référence pour cet utilisateur (1), et la comparaison (S44) de chaque PPG à chacun des modèles d'utilisateur accédés comprend la comparaison (S44) du vecteur de test avec le ou les vecteurs de référence, de préférence en calculant les distances entre, d'une part, le vecteur de test et, d'autre part, le ou les vecteurs de référence.

9. Procédé selon l'une quelconque des revendications **1** à **8,** dans lequel

le procédé comprend en outre la mise à jour continue (S493, S494) d'un ou plusieurs modèles d'utilisateur (1), de sorte que
de nouveaux modèles d'utilisateur sont stockés (S493) dans le dispositif (10), les nouveaux modèles d'utilisateur étant basés sur ceux sélectionnés parmi les PPG correspondants, et,
de préférence, les modèles d'utilisateurs sélectionnés sont supprimés (S494) du dispositif (10).

10. Procédé selon la revendication **9,** dans lequel

le ou les modèles d'utilisateur comprennent plusieurs modèles d'utilisateur,
le procédé comprend en outre la mise à jour (S48) des statistiques sur la base d'un résultat de la comparaison de chaque PPG avec chacun des plusieurs modèles utilisateur,
les modèles utilisateur sont mis à jour (S493, S494) sur la base (S49) des statistiques mises à jour.

11. Procédé selon la revendication **10,** dans lequel
la mise à jour (S493, S494) des modèles utilisateur comprend la détermination (S491), sur la base (S49) des statistiques mises à jour, s'il convient ou non de stocker une représentation de chaque PPG en tant que nouveau modèle utilisateur et, dans l'affirmative, le stockage (S493) de cette représentation en tant que nouveau modèle dans le dispositif (10).

12. Procédé selon l'une quelconque des revendications **9** à **11,** dans lequel
la mise à jour (S493, S494) des modèles d'utilisateur comprend l'exécution (S492, S494, S496) d'un algorithme de collecte des déchets utilisant (S49) les statistiques mises à jour comme entrée, afin de supprimer (S494) un ou plusieurs des modèles d'utilisateur actuellement stockés dans le dispositif (10).

13. Procédé selon l'une quelconque des revendications **1** à **12,** dans lequel

le système informatisé est un système informatisé distant, et
la demande est reçue (S70) et traitée (S90) conformément à un ou plusieurs protocoles d'authentification d'une spécification d'authentification logique et/ou d'une spécification d'authentification physique.

14. Système biométrique comprenant un dispositif biométrique (10), dans lequel le dispositif biométrique comprend

une unité de détection (100) configurée pour acquérir des signaux photopléthysmographiques ;
une interface (160) configurée pour connecter le dispositif biométrique (10) à un système informatisé (20, 30), de préférence un système informatisé distant (30), plus préférablement via un point d'accès réseau (20) ;
une unité de traitement (105) configurée pour prendre des mesures selon le procédé de l'une quelconque des revendications **1** à **13.**

15. Produit logiciel destiné à authentifier un utilisateur (1) à l'aide d'un dispositif biométrique (10), le produit logiciel comprenant un support de stockage lisible par ordinateur sur lequel sont enregistrées des instructions de programme, les instructions de programme étant exécutables par des moyens de traitement du dispositif biométrique afin d'amener ce dernier à exécuter les étapes selon le procédé de l'une quelconque des revendications **1** à **13.**

**FIG. 1A**

**FIG. 1B**

FIG. 1B

FIG. 2

FIG. 3

FIG. 4A

FIG. 4B

FIG. 5A

FIG. 5B

FIG. 6A

FIG. 6B

**EP 4 423 642 B1**

30

S35: Sense user to obtain PPG signal

S36: Store PPG signal values in circular buffer

S37: Extract and distribute PPG segments

S40: PPG matching

S42: Preprocessing

S43: Extract features (obtain vector)

S44: Compute distances to reference vectors

S45: Match?

Yes

No

S46: Set first parameter to verified state

S47: Set first parameter to non-verified state

S50: Check condition

S52: Preprocessing

S53: Identify signal features

S58: Further sensor output

S54: Analyze features (heart beat)

S59: Process output

S55: Device worn?

No

Yes

S57: Set second parameter to non-confirmed state

S56: Set first parameter to confirmed state (and log value)

S60: Update causal state parameter based on first and second parameter to lock/unlock device

```
┌─────────────────────────────────┐   ┌─────────────────────────────────────────┐
│    S45: Last PPG Match?         │   │  S55: Device worn (since last PPG match)? │
└─────────────────────────────────┘   └─────────────────────────────────────────┘
      TRUE/FALSE                                      TRUE/FALSE
```

```
              ┌─────────────────────────────┐
              │          AND?               │
              └─────────────────────────────┘
```

```
      TRUE                                          FALSE
```

```
┌─────────────────────────────────────────┐   ┌─────────────────────────────────────────┐
│ S60: Set causal state parameter to "unlock" │ S60: Set causal state parameter to "lock" │
└─────────────────────────────────────────┘   └─────────────────────────────────────────┘
```

FIG. 6C

FIG. 7

S37: Extract and distribute PPG chunks

**S42: Preprocessing**

S421: Remove DC

S422: Filter

S423: Normalize

S424: MA removal

S425:Peak detection

S426: False peak removal

S427: Segmentation

S43: Feature extraction with quantized model
(vector prediction)

S44: Compute distances
to stored user templates

S45: min(distances)
< t?

Yes

No

S46:User verified

S47: Not verified

FIG. 8

FIG. 9

**Continual updates**

S44: Compute distances to reference vectors

S48: Update statistics

S49: Analyze statistics

S491: Agreement fades?

Yes → S493: Store new user template based on last sucessfully matched vector of extracted features

No → S495: No action

**Garbage collection**

S492: Redundant/ useless template(s)?

Yes → S494: Delete user template(s)

No → S496: No action

FIG. 10A

FIG. 10B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015135310 A1

**Non-patent literature cited in the description**

- TrueHeart: Continuous Authentication on Wrist-worn Wearables Using PPG-based Biometrics. **ZHAO TIANMING et al.** IEEE INFOCOM 2020 - IEEE CONFERENCE ON COMPUTER COMMUNICA-TIONS. IEEE, 06 July 2020, 30-39

- Your Heart Won't Lie PPG-based Continuous Authentication on Wrist-worn Wearable Devices. **ZHAO TIANMING et al.** PROCEEDINGS OF THE 2018 ACM INTERNATIONAL CONFERENCE ON INTERACTIVE SURFACES AND SPACES. ACM, 15 October 2018, 783-785